# EUROPEAN PATENT SPECIFICATION

(11) **EP 2 925 241 B1**
(45) Date of publication and mention of the grant of the patent: **20.02.2019**
(21) Application number: 13858635.9
(22) Date of filing: 26.11.2013
(51) Int. Cl.: A61B 17/3205, A61B 17/22, A61B 90/00, A61B 17/16, A61B 17/32

(54) **MEMS DEBRIDER DRIVE TRAIN**
ANTRIEBSSTRANG FÜR MEMS-DEBRIDER
TRANSMISSION DE DÉBRIDEUR À SYSTÈME MICROÉLECTROMÉCANIQUE

(30) Priority: 29.11.2012 US 201261731440 P; 15.03.2013 US 201313843462
(43) Date of publication of application: 07.10.2015
(73) Proprietor: Microfabrica Inc., Van Nuys, CA 91406 (US)
(72) Inventor: SCHMITZ, Gregory P., Van Nuys, CA 91406 (US); LEGUIDLEGUID, Ronald, Van Nuys, CA 91406 (US); WU, Ming-Ting, Van Nuys, CA 91406 (US); PEREA, Juan Diego, Van Nuys, CA 91406 (US); ARCENIO, Gregory B., Van Nuys, CA 91406 (US)
(74) Representative: Potter Clarkson
(86) International application number: PCT/US2013/071874
(87) International publication number: WO 2014/085389

(56) References cited:
- EP-A2- 0 925 857
- EP-B1- 1 026 996
- US-A- 1 179 910
- US-A- 4 197 645
- US-A1- 2002 058 944
- US-A1- 2003 130 662
- US-A1- 2011 230 727
- US-A1- 2012 053 606
- US-A1- 2012 053 606
- US-B1- 6 572 613

## Description

### FIELD

Embodiments of the present disclosure relate to micro-scale and millimeter-scale tissue debridement devices that may, for example, be used to remove unwanted tissue or other material from selected locations within a body of a patient during a minimally invasive or other medical procedure, and in particular embodiments, multi-layer, multi-material electrochemical fabrication methods that are used to, in whole or in part, form such devices.

### BACKGROUND

Debridement is the medical removal of necrotic, cancerous, damaged, infected or otherwise unwanted tissue. Some medical procedures include, or consist primarily of, the mechanical debridement of tissue from a subject. Rotary debrider devices have been used in such procedures for many years.

Some debrider devices with relatively large dimensions risk removing unintended tissue from the subject, or damaging the unintended tissue. There is a need for tissue removal devices which have small dimensions and improved functionality which allow them to more safely remove only the desired tissue from the patient. There is also a need for tissue removal devices which have small dimensions and improved functionality over existing products and procedures which allow them to more efficiently remove tissue from the patient.

Prior art tissue removal devices often remove tissue in large pieces, having dimensions well over 2mm. The tissue pieces are removed through an aspiration lumen typically 3.5 to 5 mm in diameter. Since the tissue pieces being removed commonly have dimensions that are 1 to 2 lumen diameters in length, the tissue pieces can often clog the tissue removal lumen.

One portion of the body in which tissue can be removed to treat a variety of conditions is the spine area. Tissue removal devices for the spine are needed that can be produced with sufficiently small dimensions and/or that have increased performance over existing techniques. For example, a herniated disc or bulging disc can be treated by performing a discectomy, e.g. by removing all or part of the nucleus pulposus of the damaged disc. Such procedures may also involve a laminotomy or laminectomy wherein a portion or all of a lamina may be removed to allow access to the herniated disc. Artificial disc replacement (total or partial) is another example of a procedure which requires the removal of all or a portion of the disc, which is replaced with an artificial device or material.

Tissue removal devices are needed which can be produced with sufficient mechanical complexity and a small size so that they can both safely and more efficiently remove tissue from a subject at a high removal rate, and/or remove tissue in a less invasive procedure and/or with less damage to adjacent tissue such that risks are lowered and recovery time is improved.

US 2012/053606 A1 discloses a medical device for removing tissue from a subject

### SUMMARY OF THE DISCLOSURE

According to the present invention, there is provided a medical device according to claim 1. Preferred embodiments of the invention are provided in the dependent claims.

In some embodiments, the two separate spur gears of the first drive gear train may be arranged in a symmetrical fashion relative to the two separate spur gears of the second drive gear train.

In some embodiments, the tissue cutter assembly is fabricated separately from the distal housing and subsequently assembled therewith. The tissue cutter assembly may be formed at least in part by an additive process, and the distal housing may be formed at least in part by a subtractive process.

In some embodiments, the elongate member includes an annular void formed between the inner drive tube and the outer tube. In these embodiments, the device is configured to have irrigation fluid flow distally through the annular void, through the tissue cutter assembly, and then carry cut tissue pieces proximally though the inner drive tube.

Also described is a medical device for removing tissue from a subject is provided with a distal housing, an elongate member, first and second rotatable members, and a first drive gear train. In these embodiments, the distal housing is configured with a tissue cutter assembly. The elongate member is coupled to the distal housing and is configured to introduce the distal housing to a target tissue site of the subject. The elongate member has an outer tube and an inner drive tube rotatably mounted within the outer tube. The inner drive tube has a crown gear located on a distal end thereof and comprises a plurality of gear teeth. The inner drive tube has an outer diameter no greater than 12mm and no smaller than 0.5mm. The first rotatable member and the second rotatable member are each rotatably mounted to the tissue cutter assembly. The first and the second rotatable members each include a plurality of disc shaped blades. Each of the plurality of blades of the first rotatable member lies in a plane
parallel to and axially offset from a plane of another of the blades of the first rotatable member. Each of the plurality of blades of the first and the second rotatable members is directly adjacent to at least one parallel surface and positioned to partially overlap the adjacent parallel surface such that tissue may be sheared between the adjacent, overlapping blades and parallel surfaces, and such that the first and the second rotatable members are configured to rotate in opposite directions to direct tissue into an interior portion of the distal housing. The first drive gear train is coupled between the crown gear and the first rotatable member. The first drive gear train includes a first spur gear having a plurality of gear teeth arranged to mesh with the gear teeth of the crown gear. The first spur gear is configured to rotate about an axis that is not parallel to an axis of rotation of the crown gear. The crown gear teeth have a convex profile and the first spur gear teeth have a concave profile.

In some of the above embodiments, the crown gear teeth have a mid-point base thickness that is greater than a base thickness of the first spur gear teeth. In some embodiments, the ratio of the first spur gear teeth mid-point base thickness to the crown gear teeth mid-point base thickness is in the range of 0.6 to 0.9. In some embodiments, the ratio is about 0.76.

In some embodiments, the first drive gear train includes a second spur gear coupled between the first spur gear and the first rotatable member. In these embodiments, the second spur gear has teeth with a convex profile. The second spur gear teeth may have a mid-point base thickness that is greater than a mid-point base thickness of the first spur gear teeth. In some embodiments, the ratio of the first spur gear teeth mid-point base thickness to the second spur gear teeth mid-point base thickness is in the range of 0.4 to 0.95. In some embodiments the ratio is about 0.85. In some embodiments, a tangent to a mid-point base thickness of the teeth of the second spur gear slopes towards a tip of the teeth of the second spur gear. Alternatively, a tangent to a mid-point base thickness of the teeth of the second spur gear may slope away from a tip of the teeth of the second spur gear to create a bulging tip section of the teeth of the second spur gear to reduce backlash between the teeth of second spur gear and the teeth of the first spur gear. The ratio of the first spur gear teeth mid-point base thickness to the second spur gear teeth mid-point base thickness may be in the range of 0.6 to 0.9. In some embodiments, the ratio is about 0.77.

In some embodiments, the crown gear teeth have opposing side surfaces that taper towards a center point of the inner drive tube. Both the first and the second rotatable members may be driven by the first drive gear train. Alternatively, the device may include a second drive gear train coupled between the crown gear and the second rotatable member. In such embodiments, the second drive gear train includes a second spur gear having a plurality of gear teeth arranged to mesh with the gear teeth of the crown gear. The second spur gear is configured to rotate about an axis that is not parallel to an axis of rotation of the crown gear. The second spur gear teeth have a concave profile.

According to one embodiment, the medical device has the following features: The inner drive tube has a crown gear located on a distal end thereof and includes a plurality of gear teeth. The crown gear teeth have opposing side surfaces that taper towards a center point of the inner drive tube. The inner drive tube has an outer diameter no greater than 12mm and no smaller than 0.5mm. The first drive gear train includes a first spur gear having a plurality of gear teeth arranged to mesh with the gear teeth of the crown gear and a second spur gear having a plurality of gear teeth arranged to mesh with the gear teeth of the first spur gear. The crown gear teeth and the second spur gear teeth have a convex profile and the first spur gear teeth have a concave profile. The crown gear teeth and the second spur gear teeth have a mid-point base thickness that is greater than a base thickness of the first spur gear teeth. The second drive gear train includes a third spur gear having a plurality of gear teeth arranged to mesh with the gear teeth of the crown gear and a fourth spur gear having a plurality of gear teeth arranged to mesh with the gear teeth of the third spur gear. The fourth spur gear teeth have a convex profile and the third spur
gear teeth have a concave profile. The crown gear teeth and the fourth spur gear teeth have a mid-point base thickness that is greater than a base thickness of the third spur gear teeth.

In some embodiments, a tangent to a mid-point base thickness of the teeth of the second spur gear slopes away from a tip of the teeth of the second spur gear to create a bulging tip section of the teeth of the second spur gear to reduce backlash between the teeth of second spur gear and the teeth of the first spur gear. In these embodiments, a tangent to a mid-point base thickness of the teeth of the fourth spur gear slopes away from a tip of the teeth of the fourth spur gear to create a bulging tip section of the teeth of the fourth spur gear to reduce backlash between the teeth of fourth spur gear and the teeth of the third spur gear.

Other aspects of the disclosure will be understood by those of skill in the art upon review of the teachings herein. Other aspects of the disclosure may involve combinations of the above noted aspects of the disclosure. These other aspects of the disclosure may provide various combinations of the aspects presented above as well as provide other configurations, structures, functional relationships, and processes that have not been specifically set forth above.

### BRIEF DESCRIPTION OF THE DRAWINGS

FIGS. 1 - 3 illustrate an exemplary embodiment of a working end of a tissue removal device.
FIGS. 4A - 4G illustrate exemplary embodiments of drive mechanisms which can power the drive trains in the working end of tissue removal devices.
FIGS. 5A - 5C show another exemplary embodiment of a tissue removal device.
FIGS. 6A - 6C show an exemplary cutter head assembly 5332 that may be used with debriding device 5310, shown in FIGS. 5A - 5C.
FIGS. 7A - 7F show details of an exemplary rotor housing assembly 5420'.
FIG. 8 is a perspective view showing another exemplary embodiment of a cutter head assembly.
FIG. 9 is a perspective view showing the inner components of the cutter head assembly of FIG. 8 with solid lines and the outer components with phantom lines.
FIG. 10 is a similar view to FIG. 9 showing the cutter head assembly in a cross-sectional view taken along the longitudinal centerline of the device.
FIG. 11 is an exploded perspective view showing the components of the cutter head assembly of FIG. 8.
FIG. 12 is an enlarged perspective view showing the lug housing of the cutter head assembly of FIG. 8.
FIG. 13 is a perspective view showing the first and second drive gear trains of the cutter head assembly of FIG. 8.
FIG. 14 is a perspective view showing fluid flow paths of the cutter head assembly of FIG. 8.
FIG. 15A is a plan view showing gear teeth profiles of a pair of conventional involute gear teeth.
FIG. 15B is a plan view showing gear teeth profiles of a pair of concave and convex gear teeth.
FIG. 16A is radially inward plan view showing portions of two intermeshing gears having conventional involute gear teeth.
FIG. 16B is radially inward plan view showing portions of two intermeshing gears having concave and convex gear teeth.
FIG. 17 is a perspective view showing a first drive gear train having three gears with concave and convex gear teeth.
FIG. 18A is a perspective view showing a crown gear located on the distal end of an inner drive tube.
FIG. 18B is an end view showing the crown gear of FIG. 18A.
FIG. 19 is a perspective view showing a single gear drive train load path formed between two crown gears, each formed on an end of a drive tube.
FIG. 20 is a perspective view showing a dual load path gear drive train using spur gears, each coupled between two crown gears.
FIG. 21A is a side plan view showing a gear tooth having a concave profile.
FIG. 21B is a side plan view showing a gear tooth having a convex profile and configured to mate with the gear tooth of FIG. 21A.
FIG. 21C is a side plan view showing the gear tooth of FIG. 21A.
FIG. 21D is a side plan view showing the gear tooth of FIG. 21B.
FIG. 22A is a side plan view showing the gear tooth of FIG. 21A.
FIG. 22B is a side plan view showing a gear tooth having a convex profile and configured to mate with the gear tooth of FIG. 22A.
FIG. 22C is a side plan view showing the gear tooth of FIG. 22A.
FIG. 22D is a side plan view showing the gear tooth of FIG. 22B.
FIG. 23A is a side plan view showing the gear tooth of FIG. 21A.
FIG. 23B is a side plan view showing a gear tooth having a convex profile and configured to mate with the gear tooth of FIG. 23A.
FIG. 23C is a side plan view showing the gear tooth of FIG. 23A.
FIG. 23D is a side plan view showing the gear tooth of FIG. 23B.
FIG. 24 is an enlarged plan view showing the gear tooth of FIG. 21A.
FIG. 25 is an enlarged plan view showing the gear tooth of FIG. 21B.
FIG. 26 is an enlarged plan view showing the gear tooth of FIG. 22B.
FIG. 27 is an enlarged plan view showing the gear tooth of FIG. 23B.

### DETAILED DESCRIPTION

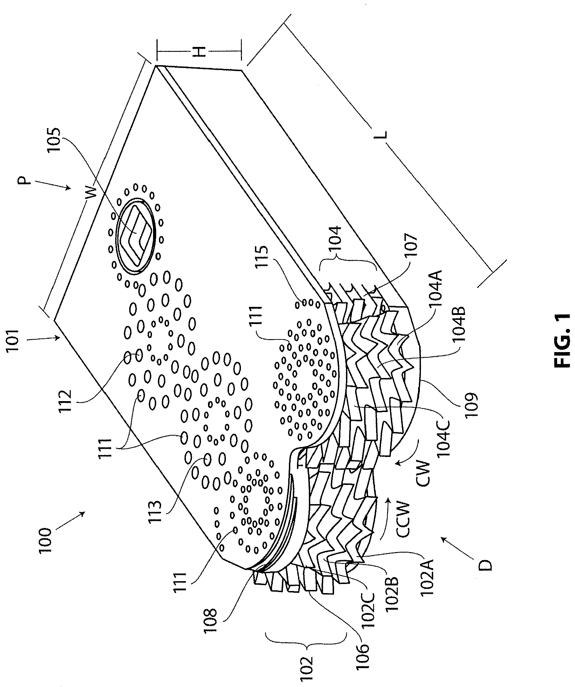

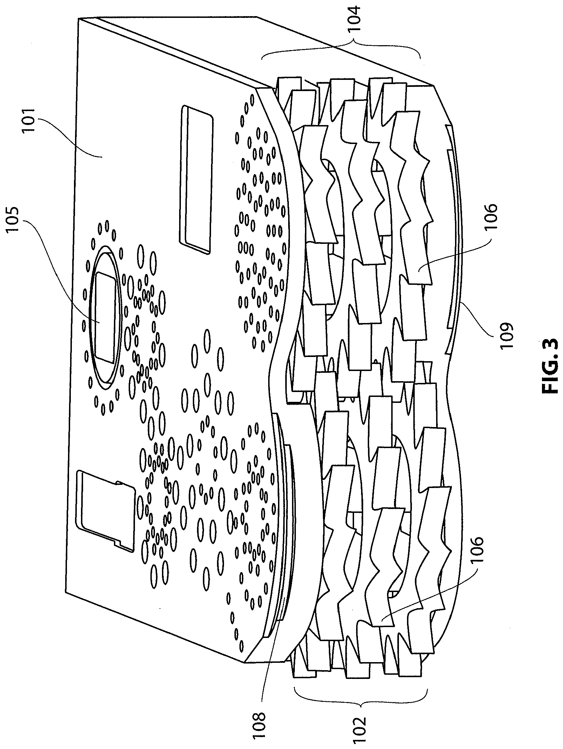

FIGS. 1 - 3 illustrate an exemplary embodiment of a working end of a tissue removal device, which can be fabricated wholly or in part by electrochemical fabrication techniques, such as those described or referenced herein. Tissue removal device working end 100 has a distal region "D" and proximal region "P," and includes housing 101 and blade stacks 102 and 104. Blade stacks 102 and 104 include a plurality of blades 102A - 102C and 104A - 104C, respectively. Three blades are shown in each stack, although the blade stacks can have one or more blades. Each of the blades includes a plurality of teeth 106 (see FIG. 3), some of which are shown projecting from housing 101 and configured to engage and process tissue. Processing tissue as used herein includes any of cutting tissue, shredding tissue, capturing tissue, any other manipulation of tissue as described herein, or any combination thereof. The working end of the device generally has a length L, height H, and width W. Housing 101 can have a variety of shapes or configurations, including a generally cylindrical shape.

In this embodiment both blade stacks are configured to rotate. The blades in blade stack 102 are configured to rotate in a direction opposite that of the blades in blade stack 104, as designated by the counterclockwise "CCW" and clockwise "CW" directions in FIG. 1. The oppositely rotating blades direct material, such as tissue, into an interior region of housing 101 (described in more detail below). In some embodiments, the blades can be made to be rotated in directions opposite to those indicated, e.g. to disengage from tissue if a jam occurs or to cause the device to be pulled distally into a body of tissue when given appropriate back side teeth configurations.

Housing 101 also includes a drive mechanism coupler 105, shown as a square hole or bore, which couples a drive train disposed in the housing to a drive mechanism disposed external to the housing. The drive mechanism, described in more detail below, drives the rotation of the drive train, which drives the rotation of the blades. The drive train disposed in the housing can also be considered part of the drive mechanism when viewed from the perspective of the blades. Drive mechanism coupler 105 translates a rotational force applied to the coupler by the drive mechanism (not shown) to the drive train disposed within housing 101.

FIG. 1 also shows release holes 111-115 which allow for removal of sacrificed material during formation of the working end.

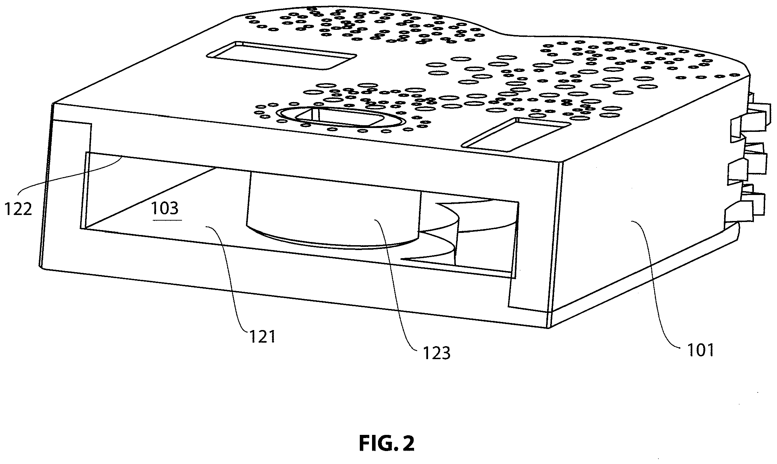

FIG. 2 shows a perspective view of the proximal end of tissue removal device working end 100. Material directed into housing 101 by the rotating blades is directed into chamber 103, wherein it can be stored temporarily or directed further proximally, as described below. A first gear train cover 121 provides for a first surface of chamber 103, while a second gear train cover 122 provides a second surface of chamber 103. FIG. 2 also shows drive mechanism coupler cover 123.

In some embodiments in which the working end 100 includes a storage chamber, the chamber may remain open while in other embodiments it may be closed while in still other embodiments it may include a filter that only allows passage of items of a sufficiently small size to exit.

FIG. 3 shows a perspective view of the distal end of the working end 100. In this embodiment the blades in stack 102 are interdigitated with the blades in stack 104 (i.e. the blade ends are offset vertically along dimension H and have maximum radial extensions that overlap laterally along the width dimension W. The blades can be formed to be interdigitated by, e.g. if formed using a multi-layer, multi-material electrochemical fabrication technique, forming each blade in stack 102 in a different layer than each blade in stack 104. If during formation portions of separately moveable blade components overlap laterally, the overlapping blades should not just be formed on different layers but should be formed such an intermediate layer defines a vertical gap between them. For example, the bottom blade in stack 102 is shown formed in a layer beneath the layer in which the bottom blade in stack 104 is formed.

When manufacturing tissue removal devices of the various embodiments set forth herein using a multi-layer multi-material electrochemical fabrication process, it is generally beneficial if not necessary to maintain horizontal spacing of component features and widths of component dimensions remain above the minimum feature size. It is important that vertical gaps of appropriate size be formed between separately movable components that overlap in X-Y space (assuming the layers during formation are being stacked along the Z axis) so that they do not inadvertently bond together and to ensure that adequate pathways are provided to allow etching of sacrificial material to occur. For example, it is generally important that gaps exist between a gear element (e.g. a tooth) in a first gear tier and a second gear tier so that the overlapping teeth of adjacent gears do not bond together. It is also generally important to form gaps between components that move relative to one another (e.g., gears and gear covers, between blades and housing, etc.). In some embodiments the gaps formed between moving layers is between about 2 µm and about 8 µm.

In some embodiments, it is desired to define a shearing thickness as the gap between elements as they move past one another. Such gaps may be defined by layer thickness increments or multiples of such increments or by the intralayer spacing of elements as they move past one another. In some embodiments, shearing thickness of blades passing blades or blades moving past interdigitated fingers, or the like may be optimally set in the range of 2 - 100 microns or some other amount depending on the viscosity or other parameters of the materials being encountered and what the interaction is to be (e.g. tearing, shredding, transporting, or the like). For example for shredding or tearing tissue, the gap may be in the range of 2 - 10 microns, or in some embodiments in the range of 4 - 6 microns.

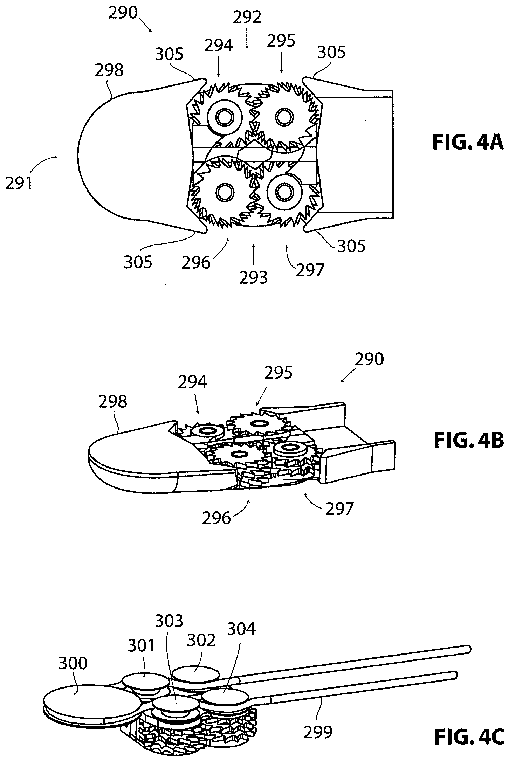

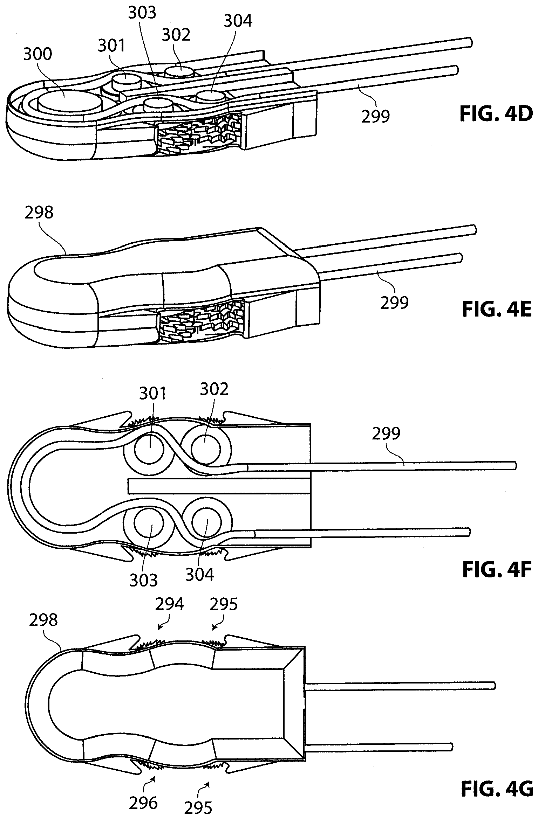

FIGS. 4A - 4G illustrate an example a of a side tissue removal working end. FIG. 4A is a top sectional view with a top portion of the housing removed, which shows working end 290 comprising housing 298 and four tissue removal elements 294-297, which are shown as blade stacks. Blade stacks 294 and 295 process tissue along one side of the housing by directing tissue in the direction of arrow 292. Blade stacks 296 and 297 process tissue along a second side of the housing by directing tissue in the direction of arrow 293. As shown in FIGS. 4A-B, blade stacks 294 and 297 each have two blades, while blade stacks 295 and 296 each have three blades. FIG. 4C shows a perspective view without housing 298 illustrating the drive mechanism for the side tissue removal device 290. The drive mechanism includes belt 299, distal pulley 300, and side pulleys 301-304. The side pulleys are coupled to the blade stacks and rotation of the side pulleys rotates the blade stacks. The belt is disposed through side pulleys 301 and 302 and around distal pulley 300 before returning through side pulleys 303 and 304. Actuating of belt 299 therefore activates all four blade stacks. In some embodiments the belt is a nitinol wire, but can be any other suitable material. FIG. 4D is a view with the top portion of the housing removed to show the internal drive mechanism. FIG. 4E shows the same view with the top on the housing. FIGS. 4F and 4G show top views of the working end shown in FIGS. 4D and 4E, respectively. Vacuum, irrigation, or a combination of the two may be used to send extracted tissue from the interior of the working end, proximally to a storage reservoir (e.g. within the working end or located outside the body of the patient on which a procedure is being performed).

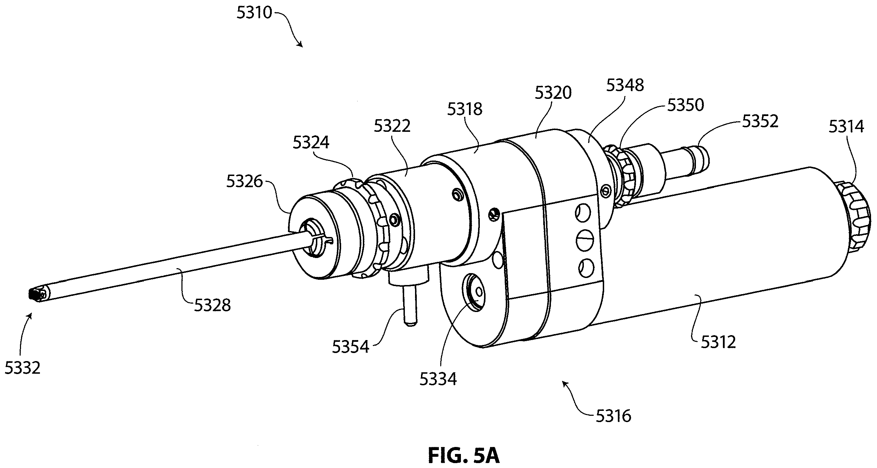

FIGS. 5A - 5C show another exemplary embodiment of a tissue removal device. Device 5310 may employ any of the cutting heads described herein, or other suitable cutting heads. In some embodiments, a double rotor shredding head is employed at the distal end of device 5310 to selectively debride tissue down to the cellular level.

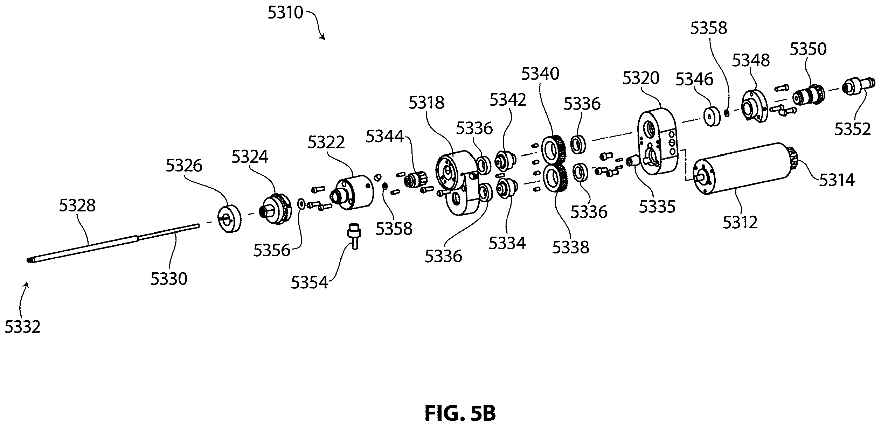

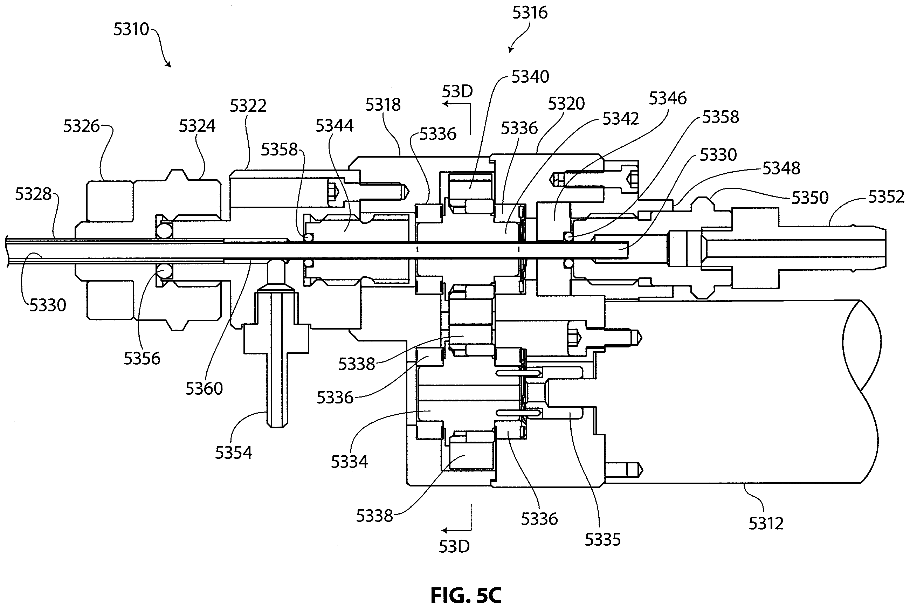

In this exemplary embodiment, handheld device 5310 includes a stepper motor 5312 at its proximal end. In other embodiments, other types of electric, pneumatic or hydraulic motors, servos, or other prime movers may be used. The proximal end of motor 5312 may be provided with a manually turnable thumbwheel 5314, as shown. In this embodiment, the distal output end of motor 5312 is provided with a housing 5316, which is made up of a front cover 5318 and a rear cover 5320. Located distally from housing 5316 are an outer shaft housing 5322, an outer shaft lock seal 5324, and a support clamp 5326. A non-rotating, outer support tube 5328 extends from within the proximal end of device 5310 towards the distal end of the device. Within support tube 5328, a rotating drive tube 5330 (best seen in FIGS. 5B and 5C) also extends from within the proximal end of device 5310 towards the distal end of the device. The support tube 5328 and inner drive tube 5330 may collectively be referred to as an introducer. A cutter head assembly 5332, subsequently described in detail, is attached to the distal end of support tube 5328.

As best seen in FIG. 5B, other components of device 5310 include motor shaft drive axle 5334, motor dog 5335, four bearings 5336, drive gear 5338, driven gear 5340, inner drive shaft axle 5342, inner shaft lock seal 5344, vacuum gland disk 5346, vacuum seal lock housing 5348, vacuum seal lock 5350, vacuum hose barb 5352, irrigation fluid hose barb 5354, outer tube o-ring 5356, and two vacuum gland o-rings 5358. Various other pins, dowels, fasteners, set screws, ball detents, shims and wave disc springs are shown in the figures without reference numerals. As will be appreciated by those skilled in this art, these non-referenced components serve to align, retain and ensure the proper functioning of the other components of exemplary device 5310.

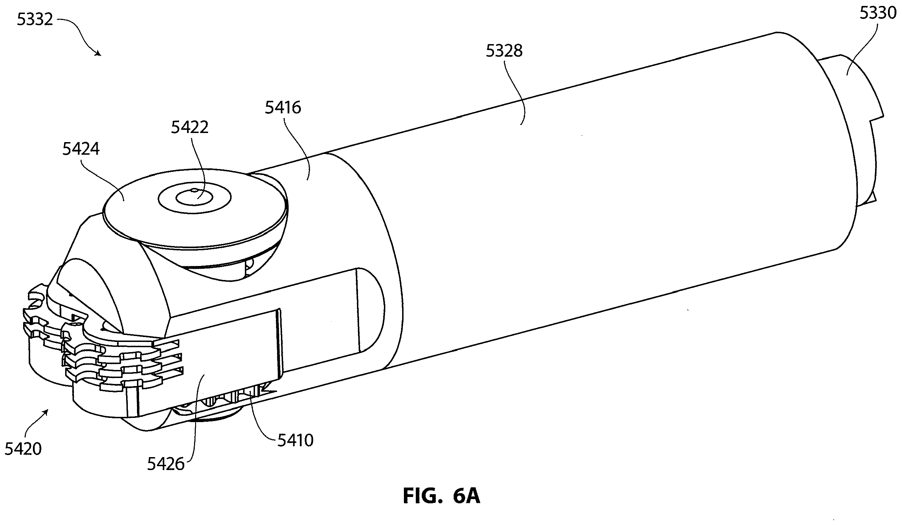

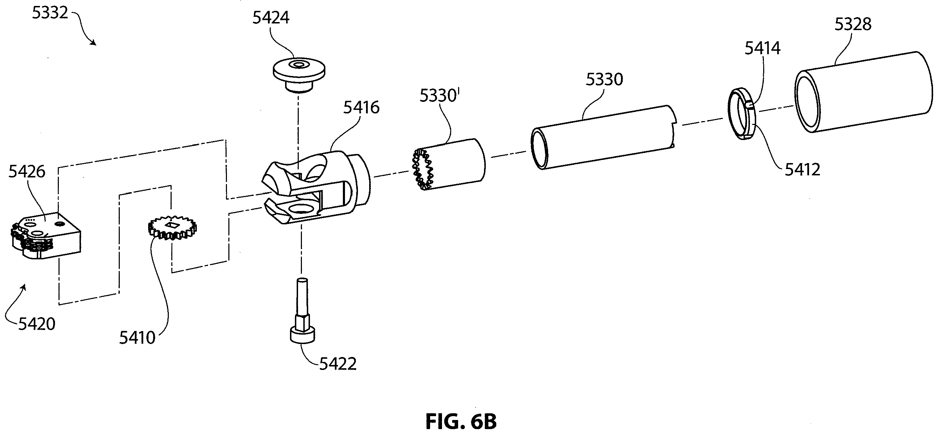

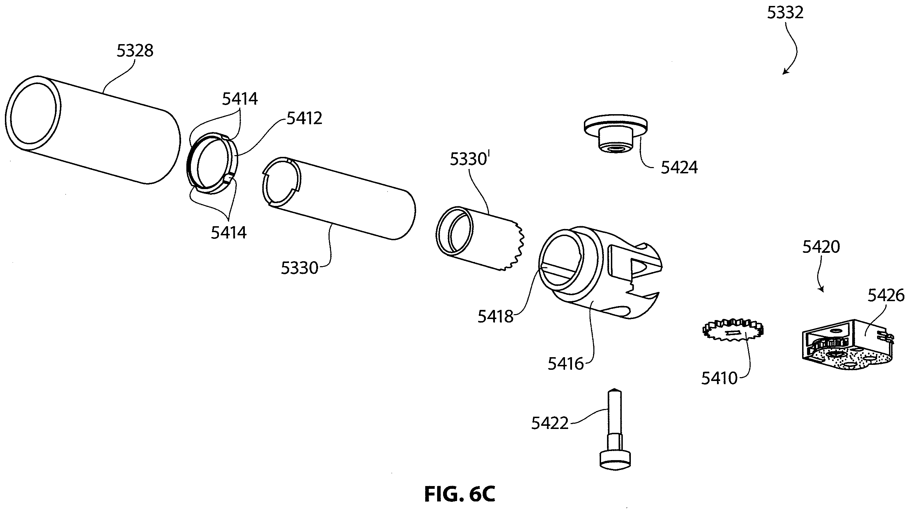

The two rotors of cutter head assembly 5332 located at the distal end of device 5310 are driven by motor 5312 through drive tube 5330 and other drive components of device 5310, as will now be described in more detail. As best seen in FIGS. 5B and 5C, a motor dog 5335 is attached to the output shaft of motor 5312. Motor dog 5335 is coupled to motor shaft drive axle 5334, which is rotatably mounted in housing 5316 with two bearings 5336. Drive gear 5338 is rigidly fixed to motor shaft drive axle 5334, and drives driven gear 5340. Driven gear 5340 is rigidly fixed to inner drive shaft axle 5342, which is rotatably mounted in housing 5316 with two bearings 5336. Inner rotating drive tube 5330 passes through the center of inner drive shaft axle 5342 and is rotatably fixed thereto. Drive tube 5330 extends from the proximal end of device 5310 to the distal end of the device through the non-rotating outer support tube 5328. The distal end of drive tube 5330 (or a separate tube 5330'attached thereto) is provided with crown teeth around its periphery, as shown in FIGS. 6B and 6C, for meshing with drive gear 5410. As drive tube 5330 is rotated about a longitudinal axis of device 5310 by motor 5312 through the above-described drive train components, it drives drive gear 5410 about an axis that is perpendicular to the longitudinal axis, as can be appreciated by viewing FIG. 6. Drive gear 5410 in turn drives other components of the cutter head assembly, and as is subsequently described in more detail.

In some embodiments motor 5312 is provided with feedback control for rotational velocity and torque. These two parameters can be used for controlling and monitoring changes in rotational velocity and the torque load. For measuring rotational velocity, an encoder may be located at one or more of the cutter rotors, at the drive motor, or at another location along the drive train between the drive motor and cutter rotors. In some embodiments, the encoder is located at or close to the rotors to avoid backlash associated with the drive train, thereby making the velocity monitoring more responsive and accurate. Encoder technologies that may be used include optical, resistive, capacitive and/or inductive measurement. To sense torque load, one or more strain gages may be located at the cutter rotors, at the drive motor, or at another location along the drive train between the drive motor and cutter rotors. Torque load may also be sensed by monitoring the current being drawn by the motor. By sensing changes in velocity and/or torque, a controller associated with device 5310 can determine that the cutter rotors are passing from one tissue type to another and take appropriate action. For example, the controller can sense when the cutter elements are passing from soft to hard tissue, from hard to medium density tissue, or from a cutting state to non-cutting state. In response to these changes, the controller and/or device 5310 can provide audio, visual and/or tactile feedback to the surgeon. In some embodiments, the controller can change the velocity, direction or stop cutter rotors from rotating in response to velocity and/or torque feedback. In one embodiment of the invention, a typical cutting rotor speed is on the order of 100 to 20,000 rotations per minute, and a typical torque load is on the order of 0.25 to 150 mN-meter. Other sensors, such as a pressure sensor or strain sensor located at the distal tip of device 5310, may also be utilized to provide feedback that tissue cutting elements are moving from one tissue type to another. In some embodiments, an impendence sensor may be located at the distal tip of the device, to sense different tissue types or conditions, and provide corresponding feedback for tissue cutting control when the tissue being cut by the cutter head changes. Such a pressure sensor feedback control arrangement can be used with types of cutting devices other than those disclosed herein.

Referring now to FIG. 5C, irrigation fluid hose barb 5354 is provided on the lower side of outer shaft housing 5322 of exemplary device 5310. Hose barb 5354, or a similar fluid line coupling, may be connected to a supply of irrigation fluid. The lumen of hose barb 5354 is in fluid communication with an internal irrigation fluid cavity 5360. Fluid cavity 5360 surrounds internal drive tube 5330, and is bounded on its proximal end by o-ring seal 5358 around drive tube 5330. Fluid cavity 5360 is bounded on its distal end by o-ring seal 5356 around outer support tube 5328. This arrangement allows drive tube 5330 to rotate, but constrains irrigation fluid delivered from hose barb 5354 to travel only through the annular space defined by the outer surface of drive tube 5330 and the inner surface of support tube 5328. Irrigation fluid may thus flow distally through the annular space to the distal end of device 5310.

As shown in FIGS. 6B, one or more drive aligner rings 5412 may be provided between outer support tube 5328 and inner drive tube 5330 along their lengths to support drive tube 5330 as it rotates. In order to allow the flow of irrigation fluid between the tubes 5328 and 5330, rings 5412 may be provided with one or more channels 5414 as shown. When the distal flow of irrigation fluid reaches the cutter head assembly 5332, it continues to flow distally into lug 5416. To enable the fluid flow, lug 5416 is provided with fluid channels 5418 located along the outer walls of its central bore, as best seen in FIG. 6C. In this embodiments, irrigation fluid passes distally between inner drive tube 5330 and lug 5416 through channels 5418 (only one channel shown in FIG. 6C). Irrigation fluid flowing distally through channels 5418 may be directed toward the outside portions of cutting elements. In this embodiment, the outside portions of cutting elements are rotating distally, away from the fluid flow, while the inside portions of cutting elements are rotating proximally, toward the center of lug 5416 and drive tube 5330.

In some embodiments, the irrigation fluid serves multiple functions. The irrigation fluid can serve to lubricate the cutting elements, drive gears, journal bearings and other components as the parts rotate. The irrigation fluid can also serve to cool the cutting elements and/or the tissue being cut, absorbing heat and carrying it away as the irrigation fluid is removed from the patient. The fluid can serve to flush tissue particles from the moving parts to prevent them from becoming clogged. The fluid can also serve to carry away the tissue portions being cut and remove them from the target tissue site. In some embodiments, the fluid comprises a saline solution. In some embodiments, the irrigation fluid is discharged from the cutting device and may be removed from the target tissue site with other, traditional aspiration means. With the current exemplary cutting device 5310, however, the irrigation fluid and/or other bodily fluids may be removed from the target tissue site by the cutting device 5310, as will now be described in detail.

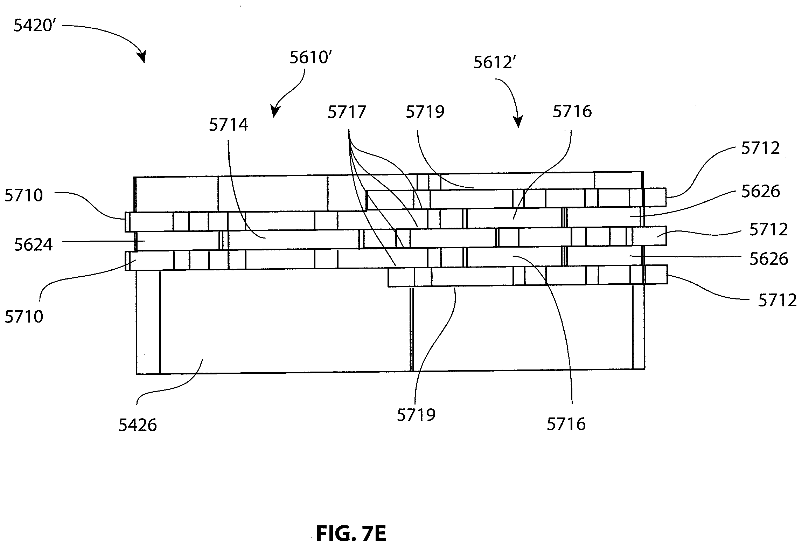

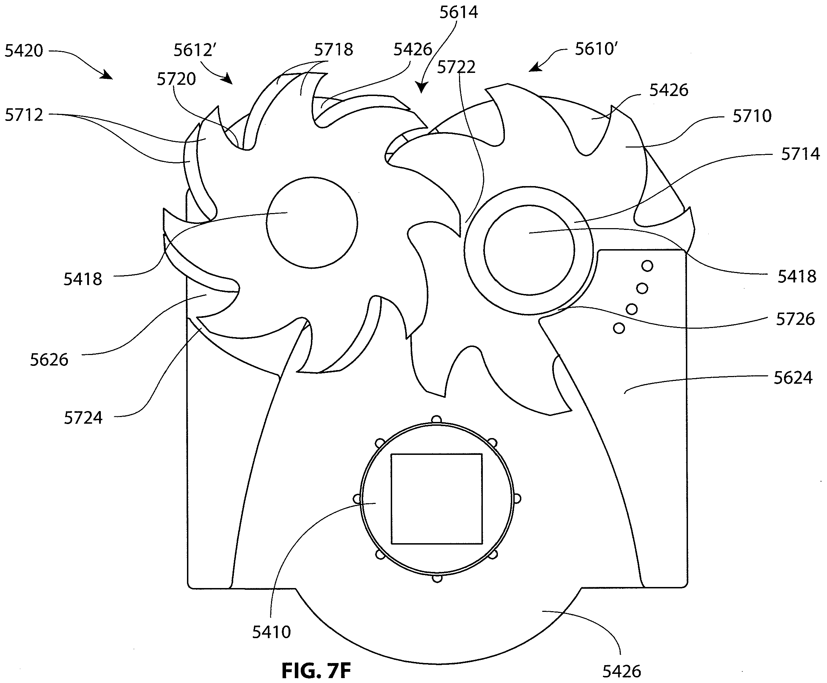

As previously described, irrigation fluid may be delivered to cutting elements and/or a target tissue site through device 5310. Exemplary device 5310 is also constructed to remove the irrigation fluid and tissue portions cut from the target tissue site through the shaft of device 5310. As can be appreciated by viewing FIG. 7F, the two interleaving stacks of cutting elements, also referred to as rotors 5610 and 5612, have an overlapping section 5614 in the center of cutter head assembly 5332. The two rotors 5610 and 5612 may be rotated in opposite directions such that each rotor engages target tissue and pulls it towards the central overlapping section 5614. In overlapping section 5614, the tissue is shredded into small pieces by the interdigitated cutting elements, as is subsequently described in more detail. The small tissue portions are generally propelled in a proximal direction by rotors 5610 and 5612, away from the target tissue site and into the cutter head assembly 5332. As can be appreciated by viewing FIG. 7F, the shredded tissue portions emerge from rotors 5610 and 5612 substantially along the central axis of lug 5416 (and therefore also the central axis of drive tube 5330. With sufficient irrigation fluid being supplied to the tissue cutting area, and sufficient aspiration being provided from the proximal end of the device, irrigation fluid around rotors 5610 and 5612 carries the cut tissue particles proximally down the center of drive tube 5330. As shown in FIG. 5C, the proximal end of drive tube 5330 is in fluid communication with hose barb 5352 located at the proximal end of device 5310. A traditional aspiration device or other suction source may be attached to device 5310 through hose barb 5352 or other suitable fluid coupling to collect the spent irrigation fluid and cut tissue portions.

In some embodiments, the cut tissues portions emerging from hose barb 5352 may be collected for testing. The tissue portions may be separated from the irrigation fluid, such as by centrifugal force, settling and/or filtering. The tissue portions may be measured to precisely determine the mass and/or volume of tissue removed. The pathology of some or all of the tissue portions may also be determined. In some embodiments, the above testing may be performed during a surgical procedure so that results of the testing may be used to affect additional stages of the procedure.

According to aspects of the disclosure, the inside diameter of drive tube 5330 may be much larger than the maximum dimension of the tissue portions traveling through it. In some embodiments, the maximum tissue dimension is less than about 2mm across. In one exemplary embodiment, the inside diameter of drive tube 5330 is about 3 mm, the outside diameter of the support tube 5328 is about 5.6 mm, and the maximum dimension of the tissue portions is about 150 microns. In another exemplary embodiment, the inside diameter of drive tube 5330 is about 1.5 mm, the outside diameter of the support tube 5328 is about 2.8 mm, and the maximum dimension of the tissue portions is about 75 microns. In other embodiments, the inside diameter of drive tube 5330 is between about 3mm and about 6mm. In some embodiments, the maximum dimension of the tissue portions is at least one order of magnitude less than a diameter of the tissue removal lumen. In other embodiments, the maximum dimension of the tissue portions is at least twenty times less than a diameter of the tissue removal lumen. In some embodiments, the maximum dimension of the tissue portions is less than about 100 microns. In other embodiments, the maximum dimension of the tissue portions is about 2 microns.

Referring now to FIGS. 6A-6C, an exemplary cutter head assembly 5332 is described in more detail. Cutter head assembly 5332 may be used with debriding device 5310, shown in FIGS. 6A - 6C. As best seen in FIG. 6B, cutter head assembly 5332 includes lug 5416, drive gear 5410, rotor housing assembly 5420, aligner pin 5422, and aligner cap 5424. Lug 5416 is provided with a cutout on its distal end for receiving rotor housing assembly 5420. Beneath the rotor housing cutout, lug 5416 has a circular recess for receiving drive gear 5410. A bore is provided in the bottom of lug 5416 for receiving the head of aligner pin 5422. When cutter head 5332 is assembled, the shank of aligner pin 5422 passes through the bore of lug 5416, through a square aperture in the center of drive gear 5410, through a bore in the proximal end of rotor housing assembly 5420, and into a large diameter bore through the top of lug 5416. Aligner cap 5424 is received with the large diameter bore in the top of lug 5416, and is fastened to aligner pin 5422 by a press fit, weld, threads, a separate fastener, or other suitable means. In this assembled arrangement, pin 5422 and cap 5424 retain rotor housing 5426 from moving longitudinally relative to the central axis of the instrument, and rotor housing 5426 and drive gear 5410 retain pin 5422 and cap 5424 from moving radially relative to the central axis of the instrument. Pin 5422 and cap 5424 spin together as a unit relative to lug 5416, and serve to align drive gear with the distal end of drive tube 5330', as previously described. Pin 5422 also serves to transmit torque from drive gear 5410 to gear 5616, which resides inside the rotor housing directly above drive gear 5410. Lug bearing 5416 forms the base of cutter head assembly 5332, shown in FIGS. 6A - 6C. As subsequently described in further detail, various different cutter heads may alternately be inserted into and secured within the slot shaped opening in the distal end of the lug bearing.

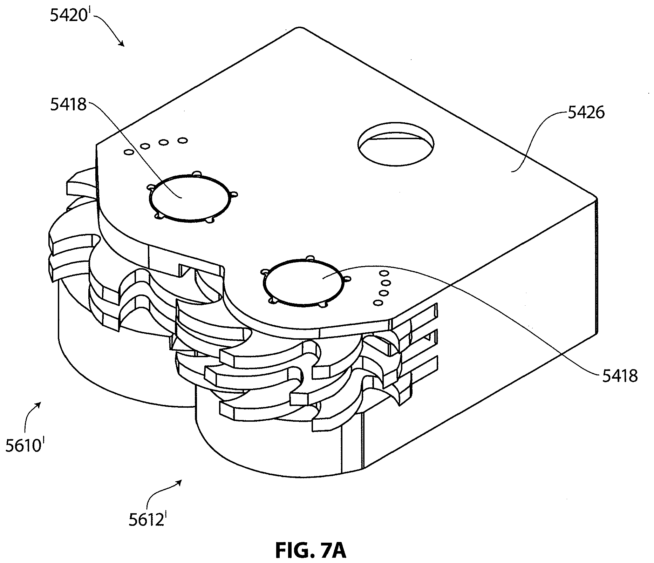

FIGS. 7A-7F show further details of an exemplary rotor housing assembly 5420'. Assembly 5420' is constructed and operates in a manner similar to assembly 5420 as previously described in reference to FIGS. 6A-6C, but has a different blade configuration. As shown in FIG. 7A, rotor housing assembly 5420' includes a pair of rotors 5610' and 5612', each rotatably mounted in rotor housing 5426 by an axle 5618. In this embodiment, rotors 5610' and 5612' are configured to rotate in opposite directions to draw tissue into a center, overlapping region where the tissue is shredded.

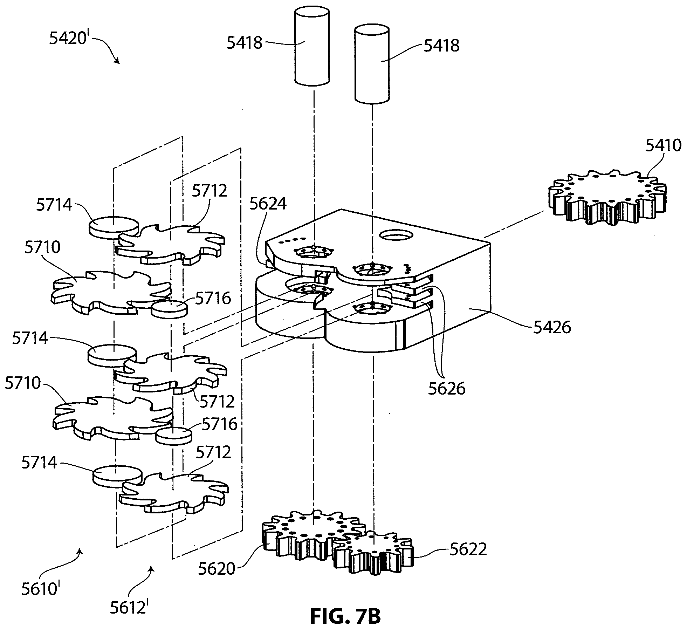

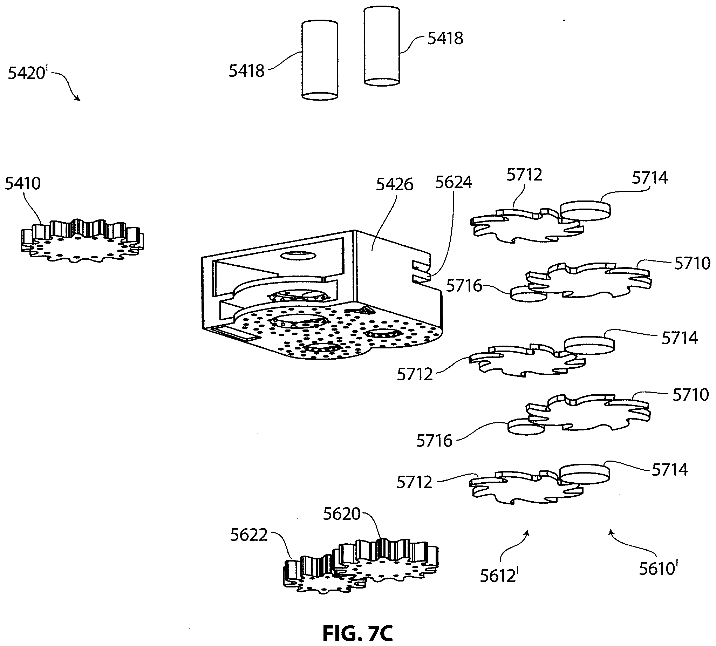

Referring to FIGS. 7B and 7C, the components of rotor housing assembly 5420' are shown. Assembly 5420' includes housing 5426, a pair of axles 5418, and gears 5410, 5620 and 5622, as previously described. Rotor 5610' includes two blades 5710 interspersed with three spacer rings 5714 on first axle 5418. Rotor 5612' includes three blades 5712 interspersed with two spacer rings 5716 on second axle 5418.

It should be noted that while rotor housing assembly 5420' is shown in an exploded format for clarity in FIGS. 7B and 7C, suggesting that the components are fabricated separately and then assembled using traditional assembly processes, this may or may not be the case, depending on the embodiment. In some embodiments, rotor assembly 5420' is assembled this way. In other embodiments, assembly 5420' may be built in layers, such as by using a MEMS fabrication processes. For example, after portions of housing 5426 and gears 5410, 5620 and 5622 are built up in layers, bottom blade 5712, bottom spacer 5714, and housing fin 5624 are formed together in one or more layers. Following this layer, bottom blade 5710, bottom spacer 5716, and bottom housing fin 5626 may be formed together in one or more layers. The process may be repeated until the entire rotors 5610' and 5612' and surrounding components are formed. A thin sacrificial layer may be formed between adjacent layers of components to separate the components from one layer from components of adjacent layers. Sacrificial material may also be formed in portions of each non-sacrificial layer to separate components on that layer, create desired voids in the finished assembly, and to provide a substrate for forming components in subsequent layers above. With such a fabrication technique, rotor 5610' may be formed as a single unitary structure interleaved with portions of rotor housing 5426, rather than separate components (i.e. axle 5418, spacers 5714, blades 5710, and gear 5620.) Similarly, rotor 5612' may be formed as a single unitary structure interleaved with portions of rotor housing 5426, rather than separate components (i.e. axle 5418, blades 5712, spacers 5716, and gear 5622.) In some embodiments, combinations of fabrication and assembly techniques may be used to create the rotor housing and/or cutter head assemblies.

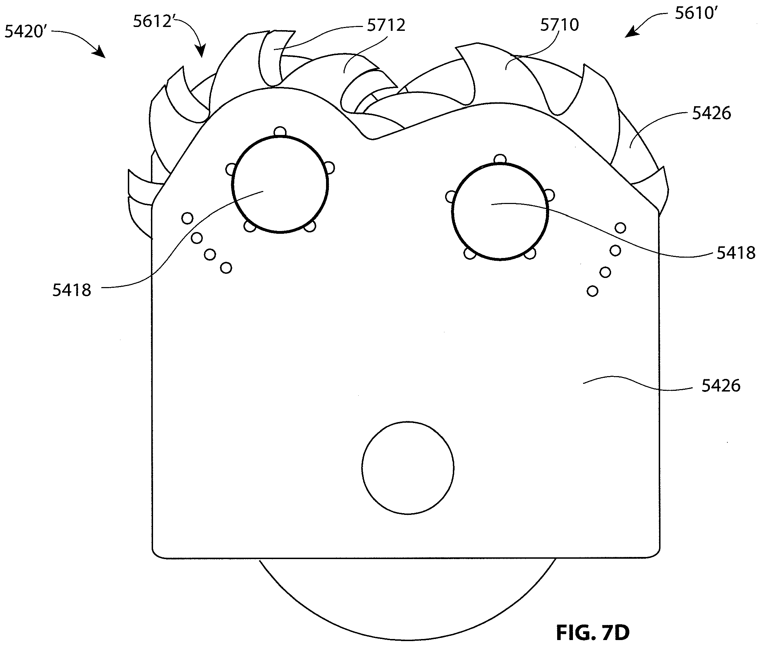

Referring to the top view shown in FIG. 7D, it can be seen that in this embodiment the axle 5418 of rotor 5612' is more distally located than axle 5418 of rotor 5610'. It can also be seen that while a top plate portion of rotor housing 5426 covers most of rotor blades 5710 and 5712, the blades protrude less from a middle and bottom plate portion of housing 5426. Further details of protruding blades and rotor characteristics are subsequently discussed in reference to FIG. 7F.

A front or distal end view is shown in FIG. 7G. As depicted in FIG. 7G, very small gaps or interference fits 5717 between overlapping blades 5710 and 5712 are desirable in some embodiments. Similarly, very small gaps or interference fits 5719 between blades 5712 and adjacent portions of rotor housing 5426 are desirable in some embodiments, as will be subsequently described in more detail.

Referring to the cross-sectional plan view of FIG. 7F, the bottom two blades 5712 of rotor 5612' and the bottom blade 5710 of rotor 5610' are shown. As shown, blades 5710 have a larger outer diameter than that of blades 5712. But because axle 5418 of rotor 5612' is located more distally than axle 5418 of rotor 5610', blades 5712 protrude more distally from the bottom of rotor housing 5426 than do blades 5710 of rotor 5610'. It can also be seen that teeth 5718 and associated troughs 5720 of blades 5712 are configured to be rotationally out of phase with those of other blades 5712 of rotor 5612'. As will subsequently be discussed in more detail, this arrangement can tune rotors 5612 to selective cut certain types of tissue and avoid cutting other types of tissue.

Various rotor gaps can be seen in FIG. 7F. For example, gap 5722 is shown between the tips of blade teeth 5718 of rotor 5612' and spacer ring 5714 / axle 5418 of opposing rotor 5610'. Gap 5724 is also shown, between the tips of blade teeth 5718 of rotor 5612' and the adjacent portion of housing 5426. Gap 5726 is also shown, between spacer ring 5714 / axle 5418 of rotor 5610' and the adjacent portion of housing 5426. In some embodiments, it is desirable to keep gaps 5722, 5724 and 5726 very small, to ensure that tissue portions/particles that pass through rotors 5610' and 5612' are first cut to a very small size, and to avoid jamming or clogging rotors 5610' and 5612'. In some embodiments, these gaps are fabricated as small interferences between the adjacent parts so that when the rotors are first rotated, the adjacent parts hit each other and wear down or burnish each other. In this manner, after a break in period, smaller interference or zero clearance fits are created between the adjacent moving parts. Gap distances that applicants believe are advantageous include less than about 20 microns, less than about 10 microns, less than about 5 microns, less than about 1 micron, substantially zero, an initial interference fit of at least 2 microns, and an initial interference fit of about 5 microns.

In operation, the cutter elements of rotor housing assembly shown in FIGS. 7A-7F serve to grab tissue from a target source, draw the tissue towards a central region between the blades, cut the tissue from the source, and morcellate the tissue in small pieces for transport away from the body. In other embodiments, separate cutter elements may be used for these various functions. For example, one blade or blades may be used to cut tissue from the source, while another blade or set of blades may be used to morcellate the cut tissue.

Components of cutter head assembly 5332, including rotor housing assemblies 5420 and 5420', may be fabricated using processes such as laser cutting/machining, photo chemical machining (PCM), Swiss screw, electro-discharge machining (EDM), electroforming and/or other processes for fabricating small parts. Wafer manufacturing processes may be used to produce high precision micro parts, such as EFAB, X-ray LIGA (Lithography, Electroplating, and Molding), and/or UV LIGA. An electrochemical fabrication technique for forming three-dimensional structures from a plurality of adhered layers is being commercially pursued by applicant Microfabrica® Inc. (formerly MEMGen Corporation) of Van Nuys, California under the name EFAB®. Such a technique may be advantageously used to fabricate components described herein, particularly rotors and associated components.

In some embodiments, the shredder's ability to selectively remove tissue is attributed to the protrusion of the rotating cutters from the housing and the design of a tooth pitch (space between the tips of adjacent teeth) of each rotor. In some embodiments, the protrusion sets the depth of the inward cut for the tips of the rotor. This inward depth controls the thickness of tissue being removed. The tooth pitch or number of teeth circumferentially about the rotor diameter provides an opening for individual tissue fibers and/or fiber bundles to be hooked, tensioned and drawn between the cutters.

From the point of view of the selected tissue, the tooth pitch and protrusion may be designed to grasp the smallest fibers or fiber bundles that are to be removed. From the point of view of the non-selected tissue, the tooth pitch may be many times smaller than the fiber or fiber bundle, and the protrusion may also be equally smaller than the fiber/bundle diameter.

As previously described, FIG. 7D shows the exemplary protrusion of blades 5710 and 5712 as viewed from the top of a rotor housing assembly 5420'. In some embodiments, the protrusion is more exposed on the top side than the bottom. In other embodiments, the cutter device has the same protrusion for both sides. Biasing the protrusion more on one side than the other can provide advantages such as cutting/shredding directionality and/or additional safety. Blade protrusion distances that applicants believe are advantageous include less than about 100 microns, less than about 10 microns, substantially flush with the housing, recessed a minimum of about 5 microns, and recessed a minimum of about 10 microns.

Tooth pitch is the distance from one tooth tip to the next tooth tip along an imaginary circle circumscribing the outer circumference of the blade. The trough diameter or depth generally is the distance between the tooth tip and the low point between the tooth tips. In many embodiments, the trough is a critical geometry component that enables tissue selectivity. Additionally, the trough opening (i.e. the distance from tooth tip to the tooth back of an adjoining tooth) can determine the size of the "window" for capturing a fiber or fiber bundle diameter.

In some embodiments, the target tissue being cut is hydrated and generally has a nominal fiber diameter of about 6 to about 9 microns. In some embodiments, the target tissue being cut is dry and generally has a nominal fiber diameter of about 5 to about 6 microns. In some embodiments, the tissue fibers are connected together in bundles having a nominal diameter of about 250 microns.

Typical dimensions in some embodiments include:
- Housing diameter: 6mm or less
- Blade diameter range: .75mm to 4mm
- Tip to Tip range: .2mm to 1mm
- Trough diameter range: 2 microns to .5mm
- Blade protrusion range: 2 microns to 2mm
The tip to tip distance is typically at least two times the trough diameter for hook type teeth.

The tissue cutting devices disclosed herein may be configured for use in a variety of procedures. An example of a cardiac application is using the inventive devices to selectively remove endocardium, with the cutting device configured to leave the underlying myocardium uncut. An example of a tissue removing application involving the esophagus includes selectively removing mucosa, leaving the submucosa. Such a therapy would be useful for treating Barrett's disease. Examples in the spinal area include selectively removing flavum, with the cutting device configured to stop removing tissue when dura is reached, leaving the dura intact. Selective removal of flavum but not nerve root is another embodiment. A cutting device constructed according to aspects of the invention can also be configured to remove flavum without cutting bone. In this embodiment, the rotor velocity could be changed and/or the cutting elements could be changed after the flavum is removed such that some bone tissue could then be removed. Examples in the neurovascular area include selectively removing cancerous tissue while not cutting adjacent blood vessel tissue or nerve tissue. In the rheumatology field, tears in labral target tissue may be selectively removed while preserving adjacent non-target tissue, such as in the hips, shoulders, knees, ankles, and small joints. In some embodiments, small teeth on the rotors can interact with micron scale fibers of cartilage, removing tissue in a precise way, much like precision machining of materials that are harder than tissue. Other target tissues that may be selectively removed by the devices and methods described herein include cartilage, which tends to be of a medium density, periosteum, stones, calcium deposits, calcified tissue, cancellous bone, cortical bone, plaque, thrombi, blood clots, and emboli.

It can be appreciated by those skilled in the art of tissue removal that soft tissue is much more difficult to remove in a small quantities and/or in a precise way than harder tissue such as bone that may be grinded or sculpted, since soft tissue tends to move or compress when being cut, rather than cut cleanly. Cutting tissue rather than removing it with a laser or other high energy device has the advantage of not overheating the tissue. This allows the tissue to be collected and its pathology tested, as previously described.

In some embodiments of the disclosure, the selective tissue cutting tool may be moved laterally along a tissue plane, removing thin swaths of tissue with each pass until the desired amount or type of tissue is removed. In some embodiments, the tool may be plunged into the target tissue in a distal direction, until a desired depth or type of tissue is reached. In any of these embodiments, the tool may cut a swath or bore that is as large as or larger than the width of the tool head. In some embodiments, the cutting elements are distally facing, laterally facing, or both.

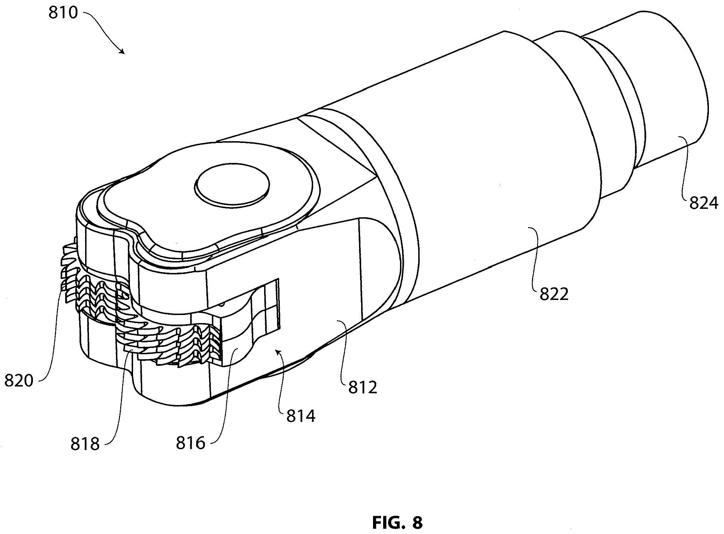

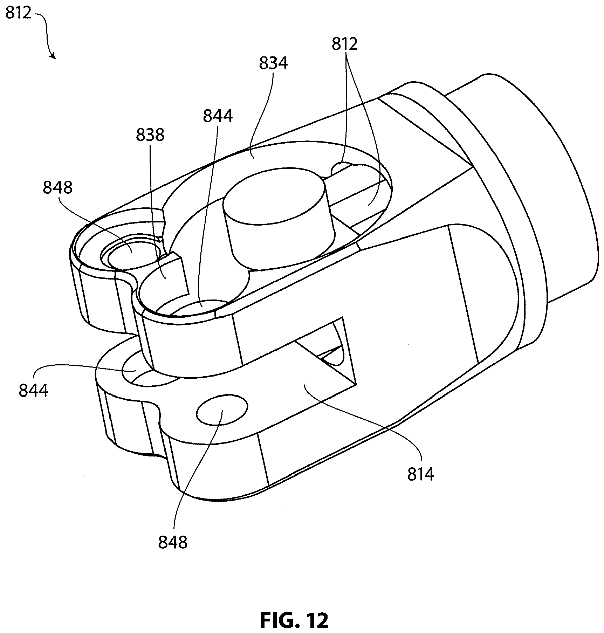

Referring to FIGS. 8 - 14, an exemplary embodiment of a cutter head assembly 810 is shown, in accordance with the present invention. As seen in FIG. 8, device 810 is similar in design to previously described cutter head assembly 5332 shown in FIGS. 6A - 6C. Similar to device 5332, device 810 includes a lug 812 provided with a transverse slot 814 (best seen in FIG. 12) at its distal end for receiving a rotor housing assembly 816. Rotor housing assembly 816 includes a first rotatable member 818 and a second rotatable member 820. The proximal end of lug 812 is configured to mate with the distal end of a bearing housing 822, or directly with the distal end of an outer support tube (not shown). Inner drive tube 824 is slidably received within the outer support tube and bearing housing 822, if provided, to drive cutter head assembly 810.

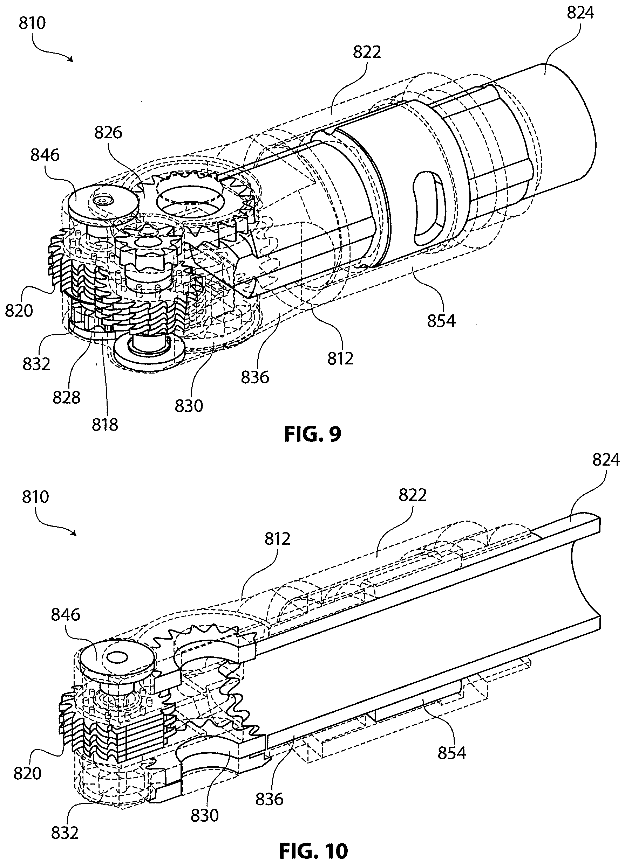

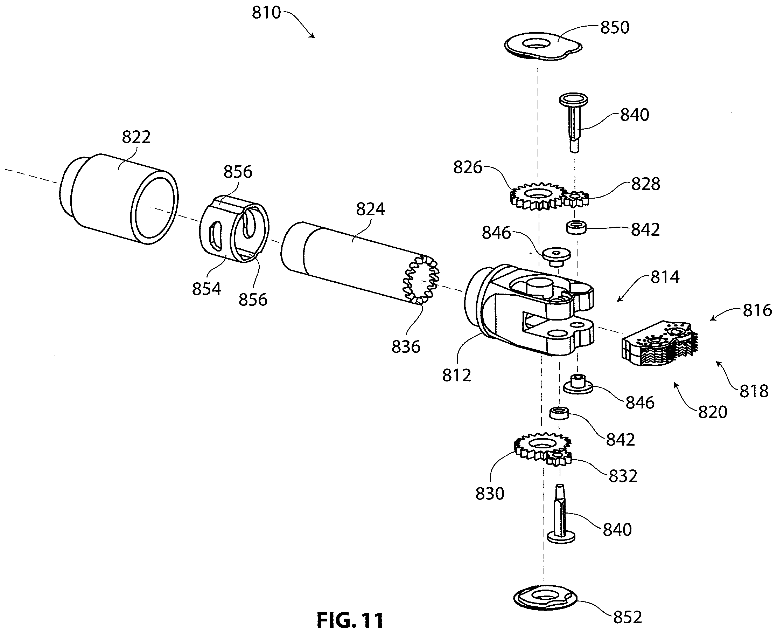

FIG. 9 is a perspective view showing the inner components of cutter head assembly 810 with solid lines and the outer components with phantom lines. FIG. 10 is a similar view to FIG. 9 but shows device 810 in a cross-sectional view taken along the longitudinal centerline of device 810. FIG. 11 is an exploded perspective view of device 810. As will now be described in reference to FIGS. 9-11, device 810 includes two separate gear drive trains. The first gear drive train is configured to drive first rotatable member 818 and the second gear drive train is configured to drive second rotatable member 820.

In this exemplary embodiment, the first gear drive train includes a large spur gear 826 and a small spur gear 828 to drive the first rotatable member 818. In a mirror image of the first gear drive train, the second gear drive train includes a large spur gear 830 and a small spur gear 832 to drive the second rotatable member 820. Large spur gear 826 is rotatably mounted within an annular recess 834 on top of lug 812, as best seen in FIG. 12. A portion of large spur gear 826 extends proximally into the interior of lug 812 to mesh with a crown gear 836 located on the distal end of inner drive tube 824, as best seen in FIG. 9. As with previous embodiments, large spur gear 826 rotates about an axis that is perpendicular to the axis of crown gear 836. Small spur gear 828 is rotatably mounted within recess 838 on top of lug 812, as best seen in FIG. 12. Small spur gear 828 is configured to mesh with and be driven by large spur gear 826. Large spur gear 830 and small spur gear 832 of the second gear drive train are received in similar recesses in the bottom of lug 812 (not shown), and operate in a manner identical to that of large spur gear 826 and small spur gear 828 of the first gear drive train.

In this exemplary embodiment, small spur gear 828 is provided with a triangular recess through its center of rotation. First rotatable member 818 is similarly provided with a triangular recess through its center of rotation. Gear pin 840 as a triangular cross-section configured to be slidably received through the triangular recesses of small spur gear 828 and first rotatable member 818. With this arrangement, small spur gear 828 rotatably drives first rotatable member 818 through gear pin 840. A bearing spacer 842, also having with a triangular recess through its center of rotation, may be provided for supporting gear pin 840. A bearing spacer recess 844 may be provided in lug 812 at the bottom of recess 838, as best seen in FIG. 12, for slidably receiving bearing spacer 842 and allowing it to rotate with respect to lug 812. This allows bearing spacer 842 to reside between the bottom of small spur gear 828 and the top of rotor housing assembly 816. A pin aligner cap 846 may also be provided for supporting gear pin 840. Pin aligner cap 846 includes a central recess configured to slidably receive a reduced diameter lower end of gear pin 840, and an outer bushing surface configured to radially support the lower end of gear pin 840 as pin aligner cap 846 spins within circular recess 848 (best seen in FIG. 12) in lug 812.

In a symmetrical fashion to the first gear drive train, large spur gear 830 and small spur gear 832 of the second gear drive train are rotatably mounted within the bottom of lug 812. A second gear pin 840, bearing spacer 842 and pin aligner cap 846 are provided to secure small spur gear 832 within similar recesses in lug 812 and to allow it to drive second rotatable member 820. Top cover 850 is provided to retain large spur gear 826 and the enlarged head of gear pin 840 of the first gear drive train, and the enlarged head of pin aligner cap 846 of the second gear drive train. In a similar fashion, bottom cover 852 is provided to retain large spur gear 830 and the enlarged head of gear pin 840 of the second gear drive train, and the enlarged head of pin aligner cap 846 of the first gear drive train. Top cover 850 and bottom cover 852 may be configured to be slightly different as shown, or may be configured to be identical so that the entire cutter head assembly 810 is symmetrical about its central axis. Top cover 850 and bottom cover 852 may be press fit, glued or welded in place, or fastened to lug 812 in another suitable manner.

A thrust bearing 854 may be slid over at the distal end of inner drive tube 824 and welded in place to axially constrain crown gear 836 of inner drive tube 824 relative to bearing housing 822 and lug 812. Thrust bearing 854 may include forward and/or rearward thrust surfaces that bear against mating surfaces within the central bore of bearing housing 822. Irrigation channels 856 (best seen in FIG. 11) may be provided on the inner and outer surfaces of thrust bearing 854 to allow a fluid to pass distally therethrough for lubricating and cooling the moving parts of device 810, and for flushing cut tissue pieces from the target tissue. Similar irrigation channels 858 (best seen in FIG. 12) may be provided axially along the central bore of lug 812.

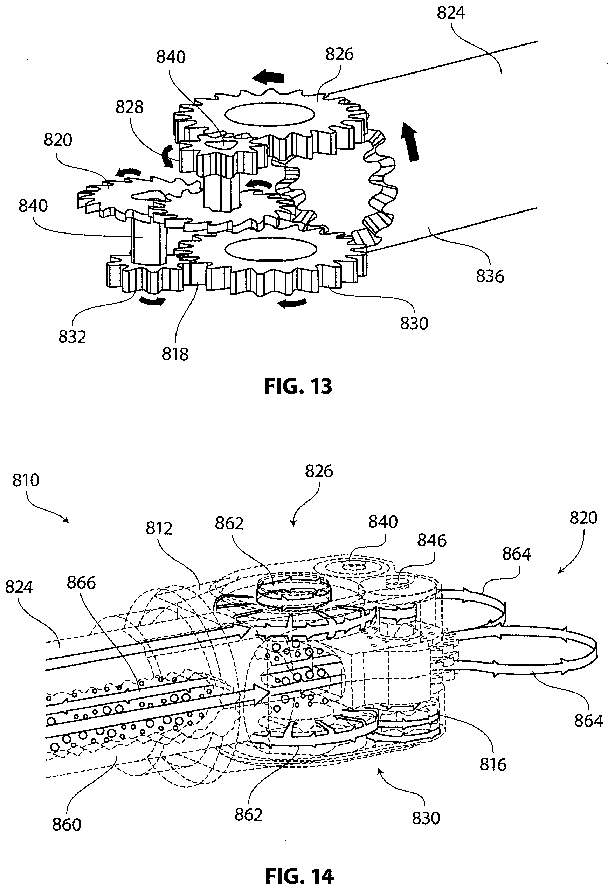

Referring to FIG. 13, operation of the first and second gear drive trains will be described. For clarity, only the basic components of the two gear drive trains and a single cutting blade from each of the two rotatable members is shown in FIG. 13. Crown gear 836 on the distal end of inner drive tube 824 may be rotated in the counterclockwise direction (as viewed when looking in the proximal direction), as shown by the arrows around inner drive tube 824. This rotates large spur gear 826 of the first gear drive train in the counterclockwise direction (as viewed from above), and rotates large spur gear 830 of the second gear drive train in the opposite, clockwise direction, as shown by the arrows around those two gears. Large spur gear 826 of the first gear drive train in turn drives small spur gear 826, gear pin 840 and the cutting blade of first rotatable member 818 in the clockwise direction, as shown by the arrows around those components. Similarly, large spur gear 830 of the second gear drive train in turn drives small spur gear 832, gear pin 840 and the cutting blade of the second rotatable member 820 in the opposite, counterclockwise direction, as shown by the arrows around those components. This arrangement drives the tissue cutting blades of the first rotatable member 818 and the second rotatable member 820 in opposite directions so that tissue may be sheared therebetween. In this embodiment, each of the two gear drive trains having two spur gears allows the drive trains to reach between crown gear 836 and its associated rotatable member 818 or 820.

Referring to FIG. 14, fluid flow through cutter head assembly 810 will be described. As with previously described embodiments, a fluid reservoir located at the proximal end of the instrument (not shown) provides fluid to the distal end of the instrument through an annular void located between inner drive tube 824 and the outer support tube and bearing housing 822 (not shown in FIG. 14). This fluid flow is depicted by arrow 860. Fluid then flows inwardly over the planar surfaces of large spur gears 826 and 830, as shown by arrows 862, and also flows around the central bores and outer teeth of those gears as shown. Fluid also flows around the central bores and outer teeth of small spur gears 828 and 832 as shown. As depicted by arrows 864, fluid also flows out of the side of rotor housing assembly 816, and flushes tissue debris from the target tissue site. This fluid returns back into the rotor housing assembly 816 through its center with the tissue debris, and up through the central bore of the inner drive tube 824, as shown by arrows 866.

The above-described arrangement provides a dual load path between inner drive tube 824 and each of the rotatable members 818 and 820. This allows critical load points, such as gear teeth and bearing surfaces, to receive half or less of the loads they might otherwise need to carry. With the very small feature sizes, high-speeds and high tissue cutting loads involved with the inventive tissue cutting devices disclosed herein, such load reduction can make the difference between a medical instrument that can reliably operate for hours and one that quickly fails. This is particularly true in instruments that need to reverse direction quickly and experience high impact loading.

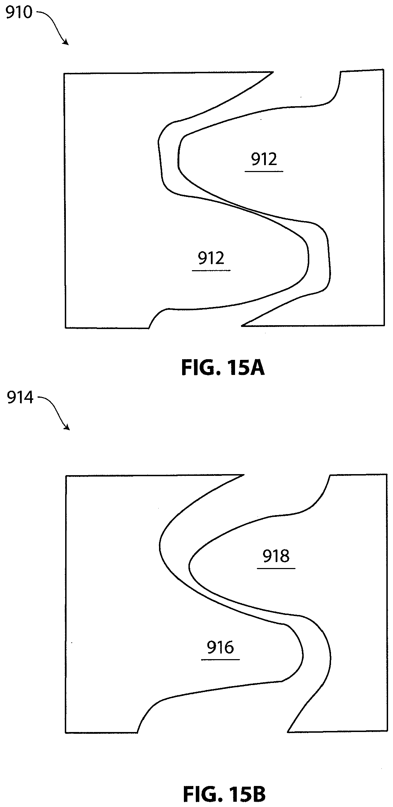

Referring now to FIGS. 15-27, further details of the previously described dual gear drive trains will be described. Some of these features may be utilized in single gear drive train arrangements and in other applications, as will also be described. Referring first to FIG. 15A, a conventional pair 910 of inter-engaging involute gear teeth 912 is shown. These involute gear teeth may be located on crown gear 836, large spur gears 826, 830, and/or on small spur gears 828, 832. The Applicants of this application have discovered however, that by modifying these gear teeth profiles, more durable gears can be created for these particular applications. In particular, Applicants have discovered that by creating an inter-meshing gear pair having one set of teeth with convex profiles, and another set of teeth with concave profiles, micro-gear trains can be created having improved operating characteristics such as higher load capabilities, higher ranges of operating speeds, reduced backlash and/or less gear wear.

FIG. 15B shows an example of an improved intermeshing gear pair 914 having one concave tooth 916 and one convex tooth 918. As can be seen by comparing FIGS. 15A and 15B, gear pair 914 can provide a greater area of contact between the intermeshing gear teeth, and can keep gear loads from being focused on the tips of the gear teeth. In some embodiments of a dual load path cutter head assembly 810 as shown in FIGS. 814, the crown gear 836 and the small spur gears 828 and 832 may be provided with convex gear teeth 918, while large spur gears 826 and 830 are provided with concave gear teeth 916. In other embodiments, crown gear 836 and the small spur gears 828 and 832 may be provided with concave gear teeth 916, while large spur gears 826 and 830 are provided with convex gear teeth 918.

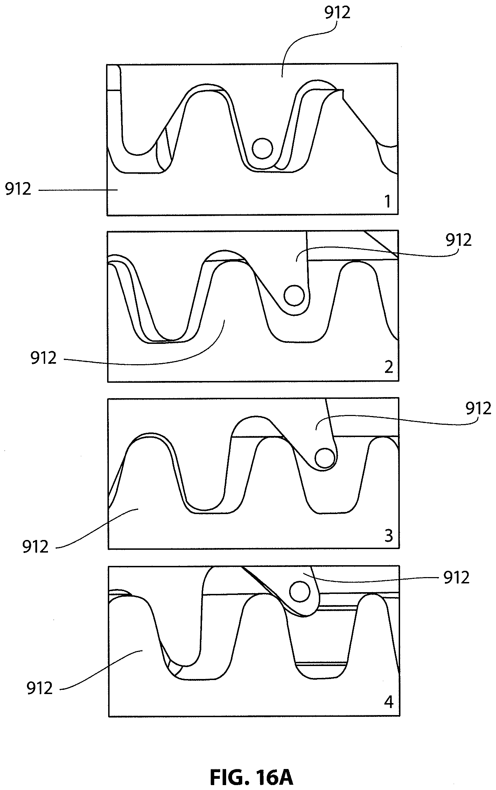

FIG. 16A shows a sequence of non-optimized rotating gears. In each of the steps 1-4, large spur gear 826 is shown above, intermeshing with crown gear 836 below. In this sequence, the gears are not properly matched. In step 1, the exiting tooth on the right of large spur gear 826 can be seen crashing into the corresponding tooth of crown gear 836.

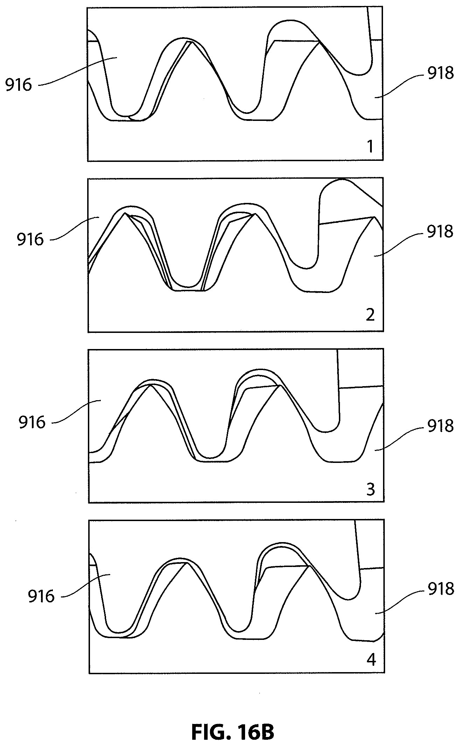

FIG. 16B shows a sequence of optimized rotating gears. Is the steps 1-4, large spur gear 826 is shown above, intermeshing with crown gear 836 below. In this sequence, the gears are properly matched according to aspects of this disclosure. In step 1, the exiting gear tooth on the right does not crash and the middle gear tooth can be seen coming into full contact with the corresponding tooth of large spur gear 826. Note that the teeth of crown gear 836 have a convex profile while the teeth of the large spur gear 826 have a concave profile.

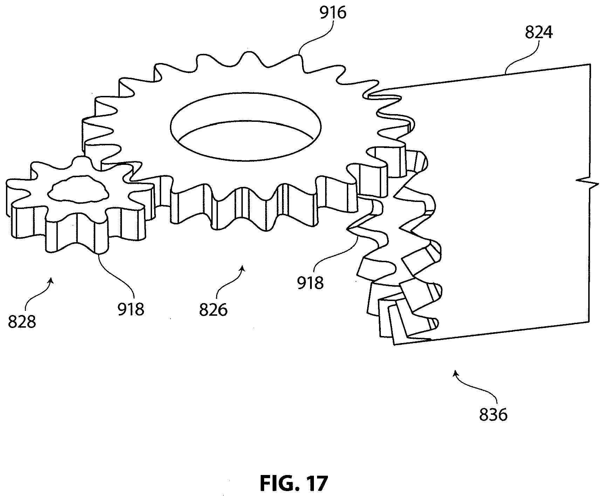

FIG. 17 shows a single gear drive train having a crown gear 836, large spur gear 826 and small spur gear 828. As shown, the teeth of crown gear 836 have a convex profile, the mating teeth of large spur gear 826 have a concave profile, and the teeth of the small spur gear 828 have a concave profile.

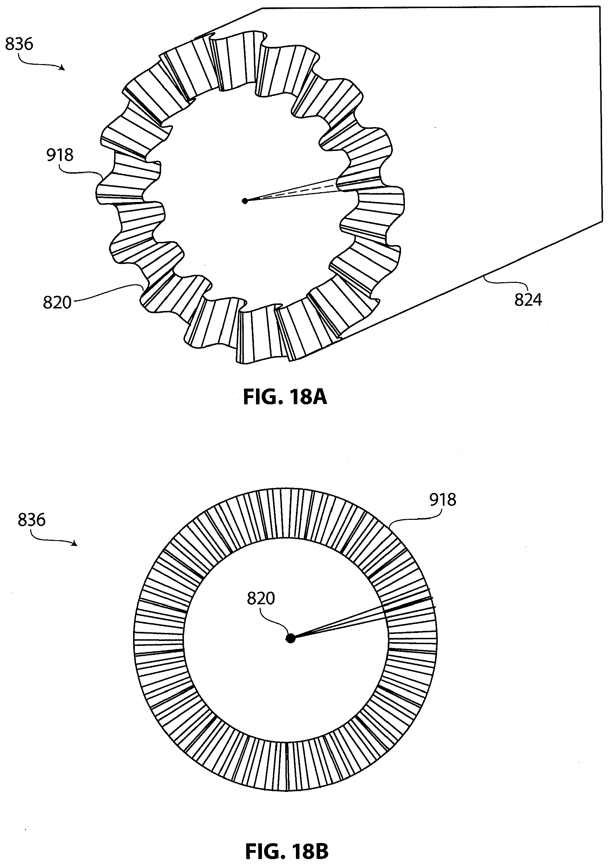

FIGS. 18A and 18B are a perspective view and an end view, respectively, showing crown gear 836 on the distal end of inner drive tube 824. As shown by the radial lines extending from the opposite surfaces of one of the gear teeth in each figure, the teeth of crown gear 836 are tapered such that each portion of a tooth surface is aligned with a point on the central axis of rotation 920 of inner drive tube 824. This inwardly tapered tooth profile assists in allowing the exiting teeth of large spur gear 826 to transition away from the teeth of crown gear 836 without crashing. These teeth may be formed with a laser cutter that cuts on center from two-dimensional data as tube 824 rotates about its central axis 920.

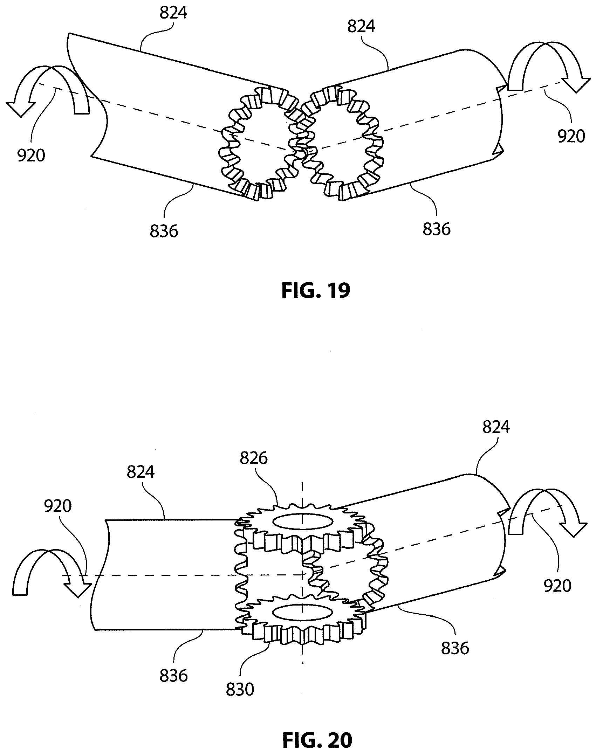

FIG. 19 shows a single gear drive train load path formed between two crown gears 836, each formed on an end of a drive tube 824. FIG. 20 shows a dual load path gear drive train using spur gears 826, each coupled between crown gears 836. Each of these arrangements allows for driving an end effector of a medical device having a sharp bend or articulation point in its shaft. This provides a significant improvement over the prior art in which only large sweeps of an instrument's shaft have been provided through the use of a gradually bent outer tube and a flexible inner tube. These new arrangements allow for instrument constructs that enable a surgeon to reach points "kicked" off to the side at the end of a straight access passage.

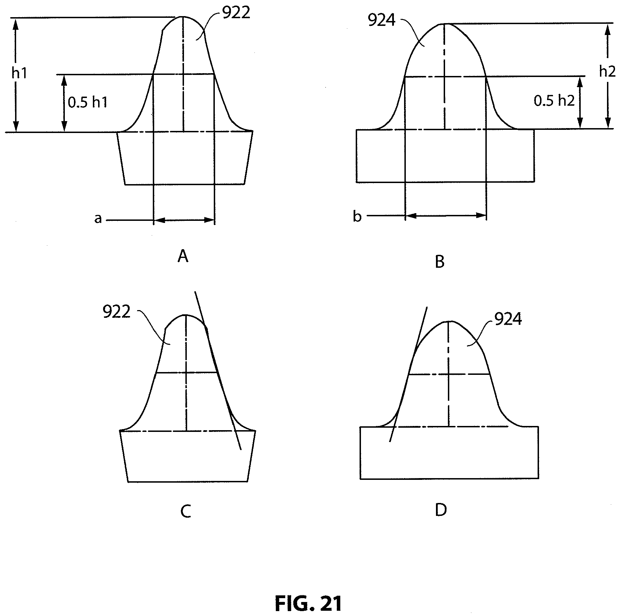

FIGS. 21-27 show additional details of the previously described gear tooth profiles. Referring first to FIG. 21, views A and C show a gear tooth 922 having a concave profile, and views B and D show a mating gear tooth 924 having a convex profile. In some embodiments, large spur gears 826 and 830 are provided with concave teeth 922, and crown gear 836 is provided with convex gear teeth 924 for driving concave teeth 922. Concave teeth 922 can be partially defined by a tooth height h1 between the tips of the gear teeth and the root circle. At a height halfway between the root circle and the tips of the teeth (0.5 h1), a base thickness or width "a" of concave tooth 922 can be defined, as shown. Similarly, convex teeth 924 can be partially defined by a tooth height h2 between the tips of the gear teeth and the root circle. At a height halfway between the root circle and the tips of the teeth (0.5 h2), a base thickness or width "b" of convex tooth 924 can be defined, as shown. In some embodiments, the ratio a/b between the base thickness of concave tooth 922 and the base thickness of convex tooth 924 is 0.76. In some embodiments, the ratio a/b is in a range of 0.6 to 0.9. Views C and D of FIG. 21 show that for both concave tooth 922 and convex tooth 924, a tangent to the tooth surface at the base thickness slopes upward and towards the tip of the tooth. Concave gear tooth profile 922 shown in views A and C of FIG. 21 can be considered concave because it has a convex to concave inflection point located between the tip of the tooth and the base thickness (i.e. on the upper half of the gear tooth).

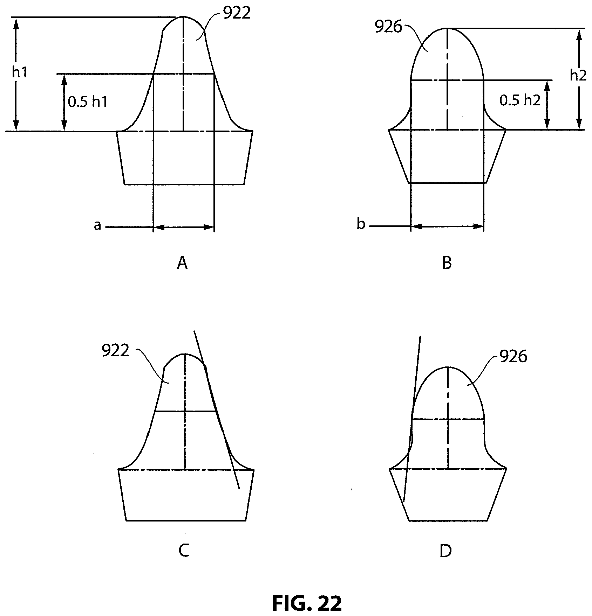

Referring now to FIG. 22, views A and C again show gear tooth 922 with its concave profile, and views B and D show a mating gear tooth 926 having a convex profile that is different than the convex profile of gear tooth 924 shown in FIGS. 21B and 21D. In some embodiments, large spur gears 826 and 830 are provided with concave teeth 922, and small spur gears 828 and 832 are provided with convex gear teeth 926, which are driven by concave teeth 922. Convex teeth 926 can be partially defined by a tooth height h2 between the tips of the gear teeth and the root circle. At a height halfway between the root circle and the tips of the teeth (0.5 h2), a base thickness or width "b" of convex tooth 926 can be defined, as shown. In some embodiments, the ratio a/b between the base thickness of concave tooth 922 and the base thickness of convex tooth 926 is 0.85. In some embodiments, the ratio a/b is in a range of 0.4 to 0.95. Views C and D of FIG. 22 show that for both concave tooth 922 and convex tooth 926, a tangent to the tooth surface at the base thickness slopes upward and towards the tip of the tooth. It can be noted that the tangent line shown in FIG. 22D for tooth 926 has a steeper angle than the tangent line shown in FIG. 21D for tooth 924.

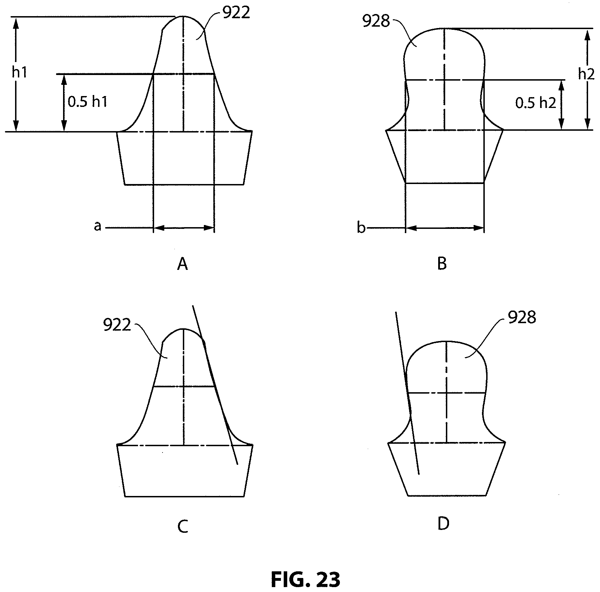

Referring now to FIG. 23, views A and C again show gear tooth 922 with its concave profile, and views B and D show a mating gear tooth 928 having a convex profile that is different than the convex profile of gear tooth 924 shown in FIGS. 21B and 21D and gear tooth 926 shown in FIGS. 22B and 22D. In some embodiments, large spur gears 826 and 830 are provided with concave teeth 922, and small spur gears 828 and 832 are provided with convex gear teeth 928, which are driven by concave teeth 922. Convex teeth 928 can be partially defined by a tooth height h2 between the tips of the gear teeth and the root circle. At a height halfway between the root circle and the tips of the teeth (0.5 h2), a base thickness or width "b" of convex tooth 928 can be defined, as shown. In some embodiments, the ratio a/b between the base thickness of concave tooth 922 and the base thickness of convex tooth 928 is 0.77. In some embodiments, the ratio a/b is in a range of 0.6 to 0.9. The View C of FIG. 23 shows that for concave tooth 922, a tangent to the tooth surface at the base thickness slopes upward and towards the tip of the tooth, but view D of FIG. 23 shows that for convex tooth 928, a tangent to the tooth surface at the base thickness slopes upward and away from the tip of the tooth. The more bulbous top portion of gear tooth 928 provides for increased anti-backlash between gear teeth 928 and 922. Convex gear tooth profile 928 shown in views B and D of FIG. 23 cannot be considered concave because it does not have a convex to concave inflection point located between the tip of the tooth and the base thickness (i.e. on the upper half of the gear tooth). On the contrary, such an inflection point only occurs between the base thickness and the root of the tooth (i.e. on the lower half of the gear tooth).

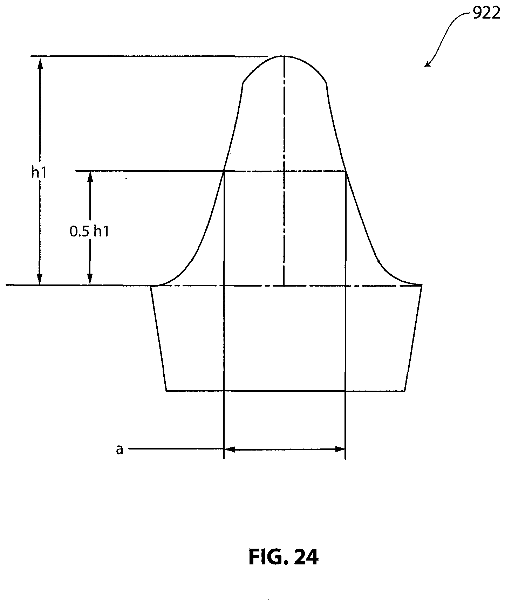

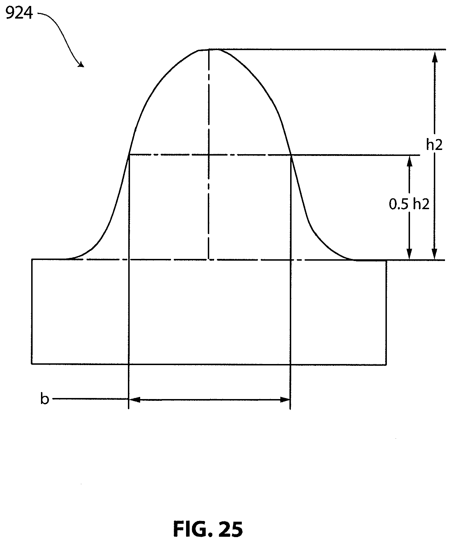

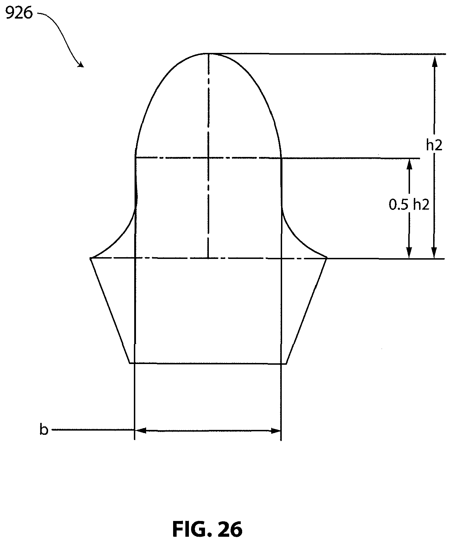

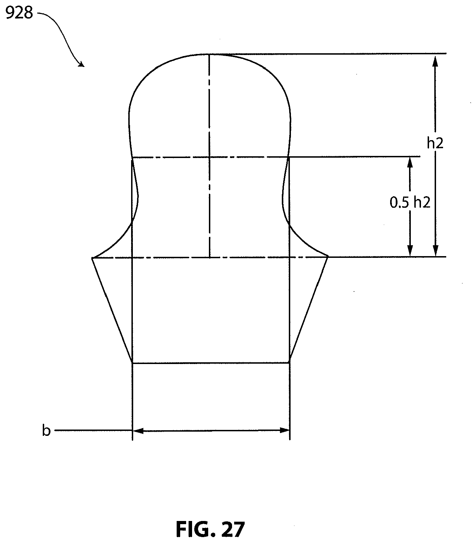

FIG. 24 is an enlarged view of concave gear tooth 922 of FIG. 21A. FIG. 25 is an enlarged view of convex gear 924 of FIG. 21B. FIG. 26 is an enlarged view of convex gear 926 of FIG. 22B. FIG. 27 is an enlarged view of convex gear 928 of FIG. 23B.

As described above, in some embodiments the gear train comprises a first convex gear driving a first concave gear, which in turn drives a second convex gear. In other embodiments, the second convex gear in such a gear train can drive a second concave gear, or the second convex gear can be omitted leaving just the first convex gear driving the first concave gear. Similarly, in some embodiments the gear train comprises a first concave gear driving a first convex gear, which in turn drives a second concave gear. In other embodiments, the second concave gear in such a gear train can drive a second convex gear, or the second concave gear can be omitted leaving just the first concave gear driving the first convex gear.

In view of the teachings herein, many further embodiments, alternatives in design and uses of the embodiments of the instant invention will be apparent to those of skill in the art. As such, it is not intended that the invention be limited to the particular illustrative embodiments, alternatives, and uses described above but instead that it be defined by the claims presented hereafter.

## Claims

1. A medical device (810) for removing tissue from a subject, comprising:
a distal housing configured with a tissue cutter assembly;
an elongate member coupled to the distal housing and configured to introduce the distal housing to a target tissue site of the subject, the elongate member having an outer tube, an inner drive tube (824) rotatably mounted within the outer tube to rotate about a longitudinal axis of the elongate member, the inner drive tube having a crown gear (836) located on a distal end thereof;
a first rotatable member (818) and a second rotatable member (820) each rotatably mounted to the tissue cutter assembly, the first and the second rotatable members each comprising a plurality of disc shaped blades, wherein each of the plurality of blades of the first rotatable member lies in a plane parallel to and axially offset from a plane of another of the blades of the first rotatable member, each of the plurality of blades of the first and the second rotatable members being directly adjacent to at least one parallel surface and positioned to partially overlap the adjacent parallel surface such that tissue may be sheared between the adjacent, overlapping blades and parallel surfaces and such that the first and the second rotatable members are configured to rotate and direct tissue into an interior portion of the distal housing;
**characterised by** a first drive gear train coupled between the crown gear (836) and the first rotatable member (818), the first drive gear train consisting of a first spur gear (826) and a second spur gear (828), wherein the first spur gear is configured to directly mesh with the crown gear (836) and rotate about a first axis that is perpendicular to the longitudinal axis, and the second spur gear is configured to directly mesh with the first spur gear and rotate about a second axis that is perpendicular to the longitudinal axis; and
a second drive gear train coupled between the crown gear (836) and the second rotatable member (820), the second drive gear train consisting of a third spur gear (830) and a fourth spur gear (832), wherein the third spur gear is configured to directly mesh with the crown gear (836) and rotate about a third axis that is perpendicular to the longitudinal axis, and the fourth spur gear is configured to directly mesh with the third spur gear and rotate about a fourth axis that is perpendicular to the longitudinal axis,
wherein the first and the second drive gear trains are configured to drive the first and the second rotatable members (818, 820), respectively, in opposite directions.

2. The medical device of claim 1, wherein the two separate spur gears (826, 828) of the first drive gear train are arranged in a symmetrical fashion relative to the two separate spur gears (830, 832) of the second drive gear train.

3. The medical device of claim 1, wherein the tissue cutter assembly is fabricated separately from the distal housing and subsequently assembled therewith.

4. The medical device of claim 3, wherein the tissue cutter assembly is formed at least in part by an additive process, and wherein the distal housing is formed at least in part by a subtractive process.

5. The medical device of claim 1, wherein the elongate member comprises an annular void formed between the inner drive tube (824) and the outer tube, and wherein the device is configured to have irrigation fluid flow distally through the annular void, through the tissue cutter assembly, and then carry cut tissue pieces proximally though the inner drive tube.

## Patentansprüche

1. Medizinische Vorrichtung (810) zum Entfernen von Gewebe von einem Subjekt, umfassend:
ein distales Gehäuse, das mit einer Gewebeschneideanordnung konfiguriert ist;
ein längliches Element, das mit dem distalen Gehäuse gekoppelt und konfiguriert ist, um das distale Gehäuse in eine Zielgewebestelle des Subjekts einzuführen, wobei das längliche Element ein äußeres Rohr, ein inneres Antriebsrohr (824), das drehbar innerhalb des äußeren Rohres montiert ist, um sich um eine Längsachse des länglichen Elements zu drehen, aufweist, wobei das innere Antriebsrohr eine Zahnscheibe (836) aufweist, die an einem distalen Ende davon angeordnet ist;
ein erstes drehbares Element (818) und ein zweites drehbares Element (820), die jeweils drehbar an der Gewebeschneideanordnung montiert sind, wobei das erste und das zweite drehbare Element jeweils eine Vielzahl von scheibenförmigen Klingen umfassen, wobei jede der mehreren Klingen des ersten drehbaren Elements in einer Ebene parallel zu und axial versetzt von einer Ebene einer anderen der Klingen des ersten drehbaren Elements liegt, wobei jede der Vielzahl von Klingen des ersten und des zweiten drehbaren Elements direkt an mindestens eine parallele Oberfläche angrenzt und derart positioniert ist, dass sie die angrenzende parallele Oberfläche teilweise überlappt, so dass Gewebe zwischen den angrenzenden, überlappenden Klingen und parallelen Oberflächen geschnitten werden kann, und so dass das erste und das zweite drehbare Element derart konfiguriert sind, dass sie Gewebe drehen und in einen Innenabschnitt des distalen Gehäuses lenken;
**gekennzeichnet durch**
einen ersten Antriebsräderzug, der zwischen der Zahnscheibe (836) und dem ersten drehbaren Element (818) gekoppelt ist, wobei der erste Antriebsräderzug aus einem ersten Geradstirnrad (826) und einem zweiten Geradstirnrad (828) besteht, wobei das erste Geradstirnrad so konfiguriert ist, dass es direkt mit der Zahnscheibe (836) in Eingriff steht und sich um eine erste Achse dreht, die senkrecht zur Längsachse ist, und das zweite Geradestirnrad so konfiguriert ist, dass es direkt mit dem ersten Geradestirnrad in Eingriff steht und sich um eine zweite Achse dreht, die senkrecht zur Längsachse ist; und
einen zweiten Antriebsräderzug, der zwischen der Zahnscheibe (836) und dem zweiten drehbaren Element (820) gekoppelt ist, wobei der zweite Antriebsräderzug aus einem dritten Geradestirnrad (830) und einem vierten Geradestirnrad (832) besteht, wobei das dritte Geradestirnrad konfiguriert ist, um direkt mit der Zahnscheibe (836) in Eingriff zu stehen und sich um eine dritte Achse zu drehen, die senkrecht zur Längsachse steht, und das vierte Stirnrad konfiguriert ist, um direkt mit dem dritten Stirnradgetriebe in Eingriff zu kommen und um eine vierte Achse zu drehen, die senkrecht zur Längsachse ist,
wobei der erste und der zweite Antriebsräderzug konfiguriert sind, um das erste beziehungsweise das zweite drehbare Element (818, 820) in entgegengesetzte Richtungen anzutreiben.

2. Medizinische Vorrichtung nach Anspruch 1, wobei die zwei separaten Geradestirnräder (826, 828) des ersten Antriebsräderzugs in einer symmetrischen Art zu den zwei getrennten Geradestirnrädern (830, 832) des zweiten Antriebsräderzugs angeordnet sind.

3. Medizinische Vorrichtung nach Anspruch 1, wobei die Gewebeschneideanordnung getrennt vom distalen Gehäuse hergestellt und anschließend damit zusammengebaut wird.

4. Medizinische Vorrichtung nach Anspruch 3, wobei die Gewebeschneideanordnung mindestens zum Teil durch ein Additivverfahren gebildet ist, und wobei das distale Gehäuse zumindest teilweise durch ein Subtraktivverfahren gebildet ist.

5. Medizinische Vorrichtung nach Anspruch 1, wobei das längliche Element einen ringförmigen Hohlraum umfasst, der zwischen dem inneren Antriebsrohr (824) und dem äußeren Rohr gebildet ist, und wobei die Vorrichtung konfiguriert ist, um einen Spülungsflüssigkeitsstrom distal durch den ringförmigen Hohlraum, durch die Gewebeschneideanordnung aufzuweisen und dann geschnittene Gewebestücke proximal durch das innere Antriebsrohr zu tragen.

## Revendications

1. Dispositif médical (810) de prélèvement d'un tissu chez un sujet, comprenant :
un logement distal, conçu avec un ensemble de coupe tissulaire ;
un élément allongé, accouplé au logement distal et conçu pour introduire le logement distal dans un site tissulaire cible du sujet, l'élément allongé présentant un tube externe, un tube d'entraînement interne (824), monté de manière rotative dans le tube externe pour tourner autour d'un axe longitudinal de l'élément allongé, le tube d'entraînement interne présentant une couronne dentée (836) située sur une extrémité distale de celui-ci ;
un premier élément rotatif (818) et un second élément rotatif (820), chacun étant monté de manière rotative sur l'ensemble de coupe tissulaire, les premier et second éléments rotatifs comprenant chacun une pluralité de lames en forme de disque, chaque lame parmi la pluralité de lames du premier élément rotatif étant située dans un plan parallèle, et décalée axialement, par rapport à un plan d'une autre des lames du premier élément rotatif, chaque lame parmi la pluralité de lames des premier et second éléments rotatifs étant directement adjacente à au moins une surface parallèle et positionnée pour chevaucher partiellement la surface parallèle adjacente, de sorte qu'un tissu puisse être cisaillé entre les lames chevauchantes adjacentes et des surfaces parallèles, et de sorte que les premier et second éléments rotatifs soient conçus pour tourner et pour diriger un tissu dans une partie intérieure du logement distal ;
**caractérisé par**
un premier train d'engrenages d'entraînement, accouplé entre la couronne dentée (836) et le premier élément rotatif (818), le premier train d'engrenages d'entraînement étant constitué d'une première roue dentée (826) et d'une deuxième roue dentée (828), la première roue dentée étant conçue pour s'engrener directement avec la couronne dentée (836) et pour tourner autour d'un premier axe, qui est perpendiculaire à l'axe longitudinal, et la deuxième roue dentée étant conçue pour s'engrener directement avec la première roue dentée et pour tourner autour d'un deuxième axe, qui est perpendiculaire à l'axe longitudinal ; et
un second train d'engrenages d'entraînement, accouplé entre la couronne dentée (836) et le second élément rotatif (820), le second train d'engrenages d'entraînement étant constitué d'une troisième roue dentée (830) et d'une quatrième roue dentée (832), la troisième roue dentée étant conçue pour s'engrener directement avec la couronne dentée (836) et pour tourner autour d'un troisième axe, qui est perpendiculaire à l'axe longitudinal, et la quatrième roue dentée étant conçue pour s'engrener directement avec la troisième roue dentée et pour tourner autour d'un quatrième axe, qui est perpendiculaire à l'axe longitudinal,
les premier et second train d'engrenages d'entraînement étant conçus pour entraîner les premier et second éléments rotatifs (818, 820), respectivement, dans des sens opposés.

2. Dispositif médical selon la revendication 1, dans lequel les deux roues dentées (826, 828) séparées du premier train d'engrenages d'entraînement sont agencées symétriquement par rapport aux deux roues dentées (830, 832) séparées du second train d'engrenages d'entraînement.

3. Dispositif médical selon la revendication 1, dans lequel l'ensemble de coupe tissulaire est fabriqué séparément du logement distal puis assemblé avec celui-ci ultérieurement.

4. Dispositif médical selon la revendication 3, dans lequel l'ensemble de coupe tissulaire est formé au moins en partie par un processus additif, le logement distal étant formé au moins en partie par un processus soustractif.

5. Dispositif médical selon la revendication 1, dans lequel l'élément allongé comprend un vide annulaire formé entre le tube d'entraînement interne (824) et le tube externe, le dispositif étant conçu pour qu'un fluide d'irrigation s'écoule de manière distale à travers le vide annulaire, à travers l'ensemble de coupe tissulaire, puis achemine des parties de tissu coupées de manière proximale à travers le tube d'entraînement interne.
